# EUROPEAN PATENT SPECIFICATION

(11) **EP 2 027 146 B1**
(45) Date of publication and mention of the grant of the patent: **03.10.2012**
(21) Application number: 07868294.5
(22) Date of filing: 30.05.2007
(51) Int. Cl.: G01N 33/68, C07K 14/11

(54) **REPLIKIN PEPTIDES AND USES THEREOF**
REPLIKIN-PEPTIDE UND VERWENDUNGEN DAVON
PEPTIDES RÉPLIKINS ET LEURS UTILISATIONS

(30) Priority: 30.05.2006 US 808944 P
(43) Date of publication of application: 25.02.2009
(73) Proprietor: Bogoch, Samuel, Dr., New York, New York 10128 (US); Bogoch, Elenore, S., New York, New York 10128 (US); Bogoch, Samuel Winston, Oakland, CA 94609 (US); Borsanyi, Anne-Elenore Bogoch, Brookline, MA 02446 (US)
(72) Inventor: BOGOCH, Samuel, New York, New York 10028 (US); BOGOCH, Elenore, S., New York, New York 10028 (US); BOGOCH, Samuel, Winston, Oakland, CA 94609 (US); BORSANYI, Anne-Elenore, B., Brookline, MA 02446 (US)
(74) Representative: Friede, Thomas
(86) International application number: PCT/US2007/069978
(87) International publication number: WO 2008/060702

(56) References cited:
- WO-A-2006/088962
- US-A1- 2005 202 415
- US-A1- 2006 024 669

## Description

### Technical Field of the Invention

This invention relates generally to a class of peptides known as Replikins and the nucleic acids encoding such peptides. Replikins share defined structural characteristics within the amino acid sequence and the nucleotide sequences encoding those amino acid sequences. Replikin peptides have been correlated with rapid replication of viruses and organisms. Replikin Scaffolds are a sub-set of the class of Replikin peptides. Replikin Scaffolds are highly conserved in strains of influenza virus that have been associated with epidemics and have been correlated with rapid replication, virulence and host mortality. The inventors have now identified patterns of substitution of amino acids, and and the corresponding nucleotides, in highly conserved Replikins and Replikin Scaffolds in influenza virus strains. The identified patterns of substitution correlate with changes in virulence and are useful as predictors of influenza epidemics and pandemics.

### Background of the Invention

Rapid replication is characteristic of virulence in certain bacteria, viruses and malignancies. U.S. Patent Appln. Ser. No. 09/984,057, filed October 26, 2001, first described Replikins as a family of conserved amino acid and nucleic acid sequences that share a prescribed sequence structure and that are found widely across rapidly-replicating malignancies, bacteria, viruses, other organisms and replication-related proteins and correlate with rapid replication and virulence. A Replikin is an amino acid sequence, or a nucleic acid sequence encoding an amino acid sequence, wherein the amino acid sequence comprises 7 to about 50 amino acids comprising a motif consisting of at least one lysine residue located at a first terminus of the motif and at least one lysine residue or at least one histidine residue located at a second terminus of the motif, at least one lysine residue located six to ten residues from a second lysine residue, at least one histidine residue and at least 6% lysine residues.

From a proteomic point of view, the Replikin motif is derived from an algorithm based on a glioma peptide sequence present in brain glioblastoma malignancies. The Replikin algorithm observed in a peptide peculiar to glioma as compared to the healthy human genome led to the discovery of a wide class of proteins with related conserved structures and a particular function, in this case replication. Correlation of an increase in virulence with an increase in the concentration of Replikin peptides present in a protein or coded in a genome was then established in disparate diseases including influenza, HIV, cancer and tomato leaf curl virus. The presence of Replikins were then correlated with the phenomenon of rapid replication in organisms as diverse as yeast, algae, plants, malaria, influenza, the Gemini leaf curl tomato virus, HIV and cancer.

In addition to detecting the presence of Replikins in rapidly replicating organisms, it was discovered that 1) Replikin concentration (number of Replikins per 100 amino acids of a protein or encoded in a genome) and 2) Replikin compositions in specific functional states dependant on rapid replication, provide the basis for the finding that Replikins are related quantitatively as well as qualitatively to the rate of replication of the organism in which they reside. Examples of these functional proofs included the relationship found between rapid replication and virulence in glioblastoma cells, between Replikins in influenza virus and the prediction of influenza pandemics and epidemics, and the relationship between Replikin concentration and rapid replication in HIV.

Replikin sequences, and in particular the defining elements of the motif of Replikin sequences, have been found to be conserved over time in rapidly replicating viruses and organisms and in proteins related to replication. Because Replikin sequences are conserved, they provide consistent targets for detection of infectious agents. This conservation of the Replikins also provides evidence that the Replikin structure itself has a role in replication and survival in those viruses and organisms where it is present and conserved. As such, Replikins identified within infectious agents are good targets for the development of treatments and vaccines against those infectious agents.

Replikin Scaffolds are a sub-set of Replikins. Replikin Scaffold peptides have been shown to be highly conserved in infectious agents and have been correlated with rapid replication, virulence and host mortality. Replikin Scaffold peptides are Replikin peptide sequences comprising about 16 to about 30 amino acids and further comprising (1) a terminal lysine, optionally comprising an additional lysine immediately adjacent to the terminal lysine; (2) a terminal histidine and a histidine immediately adjacent to the terminal histidine; (3) a lysine 6 to 10 amino acid residues from another lysine; and (4) at least 6% lysine, wherein the Replikin Scaffold peptide is a member of a series of conserved Replikin peptides identified within individual isolates of a virus or organism across time, geographic space or epidemiological occurrence or identified in different viruses or organisms that share genetic information, for example, through genetic shift.

As discussed above, the first Replikin discovered was the glioma Replikin, which was identified in brain glioblastoma multiforme (glioma) cell protein, called malignin. *See* U.S. Patent No. 7,189,800. The glioma Replikin was not known to be present in the normal healthy human genome. An algorithm devised to search for homologues of the glioma Replikin revealed that homologues were not common in over 4,000 protein sequences. Surprisingly, however, homologues were found in all tumor viruses and in the replicating proteins of algae, plants, fungi, viruses and bacteria. *See* U.S. Patent Appln. Ser. No. 10/189,437, filed July 8, 2002. As such, the presence of Replikins across a breadth of viruses and organisms was correlated with replicating functions and rapid replication.

Upon wider review of rapidly replicating viruses and organisms and replication-related proteins, it was discovered that the number of Replikins per 100 amino acids in a genome, protein or protein fragment was correlatable with the functional phenomenon of rapid replication. *See* U.S. Patent Appln. Ser. No. 10/189,437.

The correlation between Replikin concentration and rapid replication was further demonstrated in a comparison of the amino acid sequences of influenza virus hemagglutinin protein with epidemiological data for influenza outbreaks over the past 100 years. This comparison revealed a four to ten-fold increase in the concentration of strain-specific influenza Replikins in influenza epidemics caused by one of each of the four major strains of influenza between 1902 and 2001. The four major strains of influenza are influenza B, (A)HIN1, (A)H2N2 and, (A)H3N2. It was then demonstrated that the increases in concentration were due to the reappearance of at least one specific Replikin composition from 1 to up to 64 years after its disappearance, plus the emergence of new strain-specific Replikin compositions. *See* U.S. Patent Appln. Ser. No. 10/860,050.

Replikin amino acid structures have been observed not to mutate or change to the same degree as non-Replikin amino acids. Replikin structures are conserved across time in viruses and organisms and between viruses and organisms. This conservation demonstrates the Replikin structure has importance in survival. As such, conserved Replikin structures provide new invariant targets, related to survival, that are useful for identification and for treatment of infections or malignancies.

Replikins have been shown to be conserved in a range of viruses and organisms including bacteria, viruses, plants and malignancies. Because certain structures too closely related to survival functions apparently cannot change constancy, conserved Replikin structures across bacteria, virus, plants and other organisms suggest Replikins are intimately involved in survival functions.

Consideration of whether Rcplikins are conserved or, instead, are subject to extensive natural mutation was examined by scanning the protein sequences of various isolates of foot and mouth disease virus (FMDV), where mutations in proteins of these viruses have been well documented worldwide for decades. Protein sequences of FMDV isolates were visually examined for the presence of both the entire Replikin and each of the component Replikin amino acid residues observed in a particular Replikin. Rather than being subject to extensive substitution over time as occurs in neighboring amino acids, the amino acids which comprise the Replikin structure were observed to be substituted little or not at all, that is, the Replikin structures were conserved as compared to non-Replikin sequences. Similar sequence conservation was observed in plants such as in wheat ubiquitin activating enzyme E and in trans-activator (Tat) proteins in isolates of HIV. *See* U.S. Patent Appln. Ser. No. 10/860,050.

The conservation of any structure is critical to whether that structure provides a stable invariant target to attack and destroy or to stimulate. When a structure is tied in some way to a basic survival mechanism of the organism, the structures tend to be conserved. A varying structure provides an inconstant target, which is a good strategy for avoiding attackers, such as antibodies that have been generated specifically against the prior structure and thus are ineffective against the modified form. This strategy is used by influenza virus, for example, so that a previous vaccine may be quite ineffective against the current virulent virus.

An essential component of the Replikin structure is histidine (h), which is known for its frequent binding to metal groups in redox enzymes and its probable function in providing a source of energy needed for replication. A review of Replikin sequences over time suggests the histidine structure in Replikins remains constant. As such, the Replikin structure remains an all-the-more attractive target for destruction of the replication of a virus or an organism.

Replikin-containing proteins also are associated frequently with redox functions, and protein synthesis or elongation, as well as with cell replication. The association with metal-based redox functions, the enrichment of the Replikin-containing glioma malignin concentration during anaerobic replication, and the cytotoxicity of antimalignin antibody at low concentrations (picograms/cell) all suggest that the Replikins are related to central respiratory survival functions, which have been found less often subjected to the mutations characteristic of non-Replikin amino acids. *See* U.S. Patent Appln. Ser. No. 10/860,050.

In rapidly replicating viruses and organisms, a correlation has been established between the rate of replication and the concentration of Replikin sequences present in a virus or organism. The concentration of Replikin sequences within 100 amino acids is called the Replikin Count of a virus or organism. In influenza virus, an increase in Replikin Count has been correlated with influenza epidemics over the past 100 years.

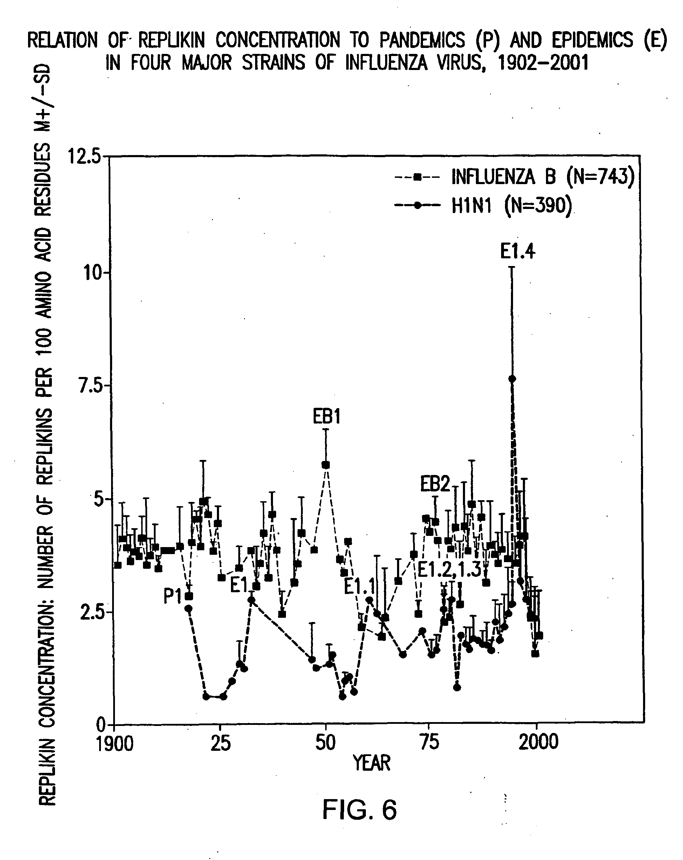

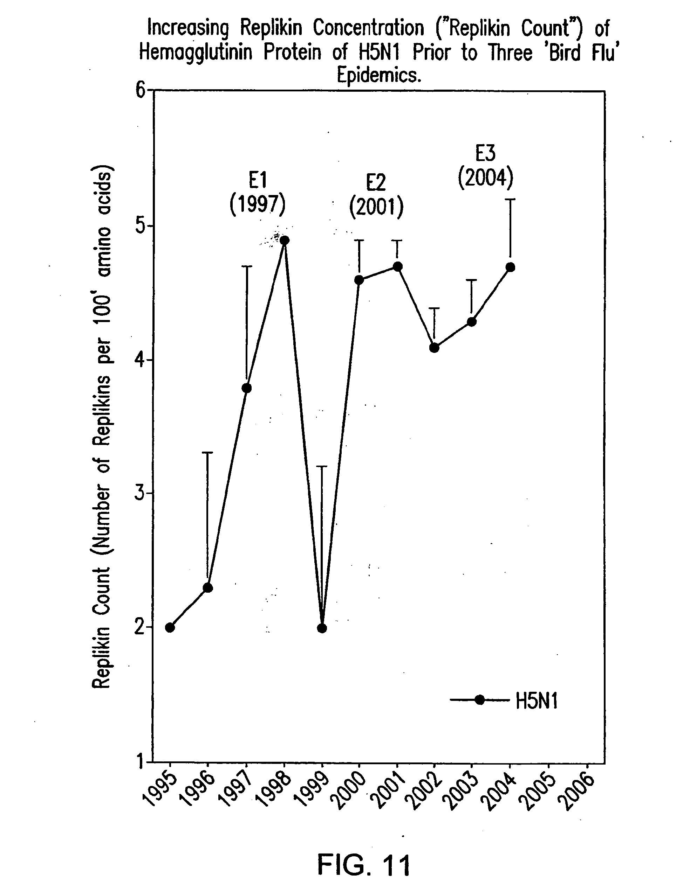

For example, a quantitative correlation of strain-specific Replikin concentration in the hemagglutinin protein with influenza epidemics and pandemics has been established (Figure 6). In each of the three influenza pandemics of the last century, H1N1, H2N2 and H3N2, the pandemic retrospectively was predicted by and correlated with an increase in the Replikin Count. Figures 6-8 and 10-11. An increase in Replikin Count has also been predictive in each of the four H5N1 epidemics, namely, epidemics in 1997, 2001, and 2003-2004 (Figure 11) and 2006. No previous correlation of influenza epidemics with strain-specific viral protein chemistry had until then been reported.

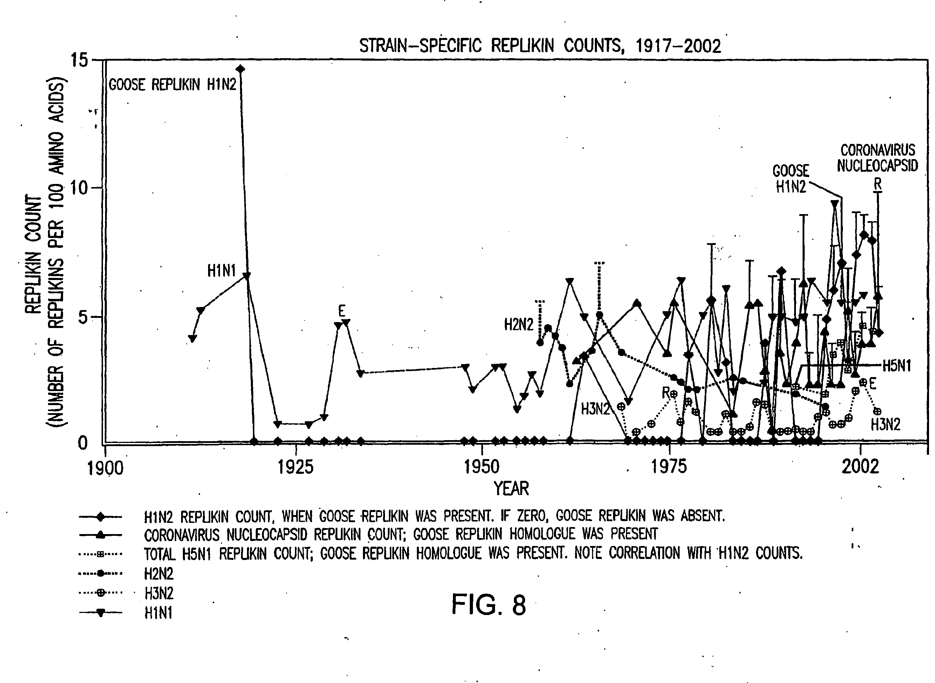

Similar to the findings of strain-specific Replikin Count increases in the influenza group one to three years prior to the occurrence of strain-specific epidemics, an increase in Replikin Count in the coronavirus nucleocapsid protein has been found to be retrospectively predictive of the SARS pandemic of 2003. Replikin Counts of the coronavirus nucleocapsid protein increased as follows: 3.1 (±1.8) in 1999; 3.9(±1.2) in 2000; 3.9 (±1.3) in 2001; and 5.1 (±3.6) in 2002. This pre-pandemic increase supports the finding that a Replikin-rich coronavirus was responsible for the SARS pandemic of 2003. (See Figure 8).

Replikin Count in the HIV virus has also been correlated with rapid replication and virulence. In HIV isolates, the slow-growing low-titer strain of HIV (NSI, "Bru," which is prevalent in early stage HIV infection) was found to have a Replikin concentration of 1.1 (+/- 1.6) Replikins per 100 amino acids, whereas the rapidly-growing high-titer strain of HIV (S1, "Lai," which is prevalent in late stage HIV infection) has a Replikin concentration of 6.8 (+/- 2.7) Replikins per 100 amino acid residues. Further, Tomato Leaf Curl Gemini virus, which has devastated tomato crops in China and in many other parts of the world, has been shown to have high Replikin Counts because of overlapping Replikins. Replikin Count in Tomato Leaf Curl Gemini virus has been observed to reach as high as 20.7 Replikins per 100 amino acids.

Replikin Scaffolds are a sub-set of the class of Replikins initially identified in strains of influenza virus. Replikin Scaffolds are highly conserved in virulent strains of influenza. Replikins and Replikin Scaffolds have been correlated with rapid replication and virulence and the presence and concentration of Replikins in emerging strains of influenza virus is now used to predict forthcoming influenza epidemics. There is a need in the art for methods of identifying small changes within a Replikin or Replikin Scaffold that result in increased virulence and host mortality. There is additionally a need in the art for conserved Replikin and Replikin Scaffold sequences that are targets for treatment of highly virulent strains of influenza. There is furthermore a need in the art for Replikin and Replikin Scaffold amino acid sequences useful for the preparation of vaccines and other therapies in emerging strains of influenza (see also US 2006/024669; WO 2006/088962)

### SUMMARY OF THE INVENTION

The invention provides a vaccine as defined in the claims, comprising isolated influenza virus Replikin peptides. In yet another aspect, the invention provides antibodies to the isolated influenza virus Replikin peptides .

It is also disclosed an isolated Replikin peptide wherein the Replikin peptide comprises about 29 amino acids. The isolated Replikin peptide may comprise the amino acid sequence KKNSTYPTIKRSYNNTNQEDLLV[S]WGIHH wherein [S] may be any amino acid other than leucine. In a preferred embodiment, [S] may be an amino acid other than leucine. In another preferred embodiment, [S] may be any hydrophobic amino acid including methionine, isoleucine, tryptophan, phenylalanine, alanine, glycine, proline or valine. In a another preferred embodiment, [S] may be a methionine or an isoleucine. In another preferred embodiment, the isolated influenza Replikin peptide is isolated from the H5N 1 strain of influenza.

The present invention also provides a vaccine comprising any one or more of the isolated sequences described above, or an antigenic subsequence thereof, or an antibody that binds to any of the isolated sequences described above, or an antigenic subsequence thereof.

In a preferred embodiment, the amino acid residue substituted in the first Replikin Scaffold as compared to the second Replikin Scaffold is five amino acid residues from the terminal histidine of the second Replikin Scaffold. In another preferred embodiment, the amino acid residue substituted in the first Replikin Scaffold as compared to the second Replikin Scaffold is any amino acid residue other than leucine. In another preferred embodiment, the amino acid residue substituted in the first Replikin Scaffold as compared to the second Replikin Scaffold is a hydrophobic amino acid other than leucine. In another preferred embodiment, the amino acid residue substituted in the first Replikin Scaffold as compared to the second Replikin Scaffold is a methionine or an isoleucine. In another preferred embodiment, the first, second and third Replikin Scaffolds comprise 29 amino acids. In yet another embodiment, the increase in virulence is indicative of a pandemic.

### BRIEF DESCRIPTION OF THE DRAWINGS

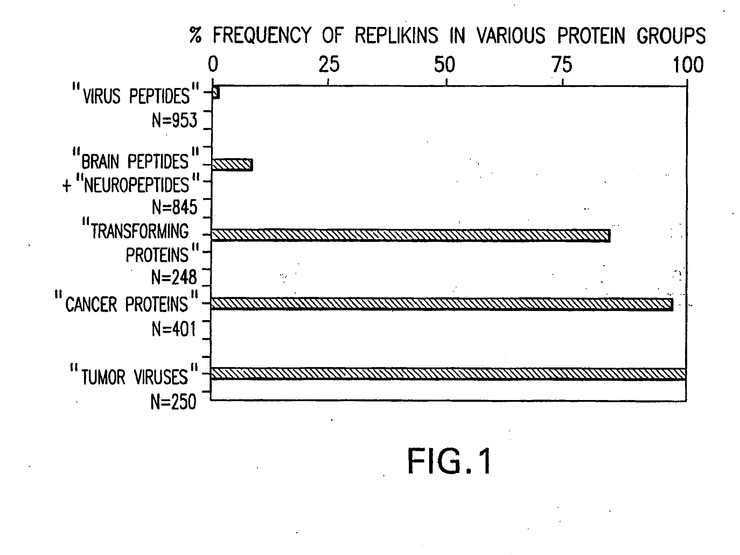

Figure 1 is a bar graph depicting the frequency of occurrence of Replikins in various organisms.

Figure 2 is a box diagram depicting an aspect of the invention wherein a computer is used to carry out a method of predicting an increase in replication, virulence or host mortality by comparing Replikin Scaffolds of at least three strains of influenza virus.

Figure 3 is a bar graph showing amount of antimalignin antibody produced in response to exposure to the recognin 16-mer.

Figure 4A is a photograph of a blood smear taken with ordinary and fluorescent light. Figure 4B is a photograph of a blood smear taken with ordinary and fluorescent light illustrating the presence of two leukemia cells. Figure 4C is a photograph of a dense layer of glioma cells in the presence of antimalignin antibody. Figure 4D and Figure 4E are photographs of the layer of cells in Figure 4C taken at 30 and 45 minutes following addition of antimalignin antibody.

Figure 4F is a bar graph showing the inhibition of growth of small cell lung carcinoma cells in vitro by antimalignin antibody.

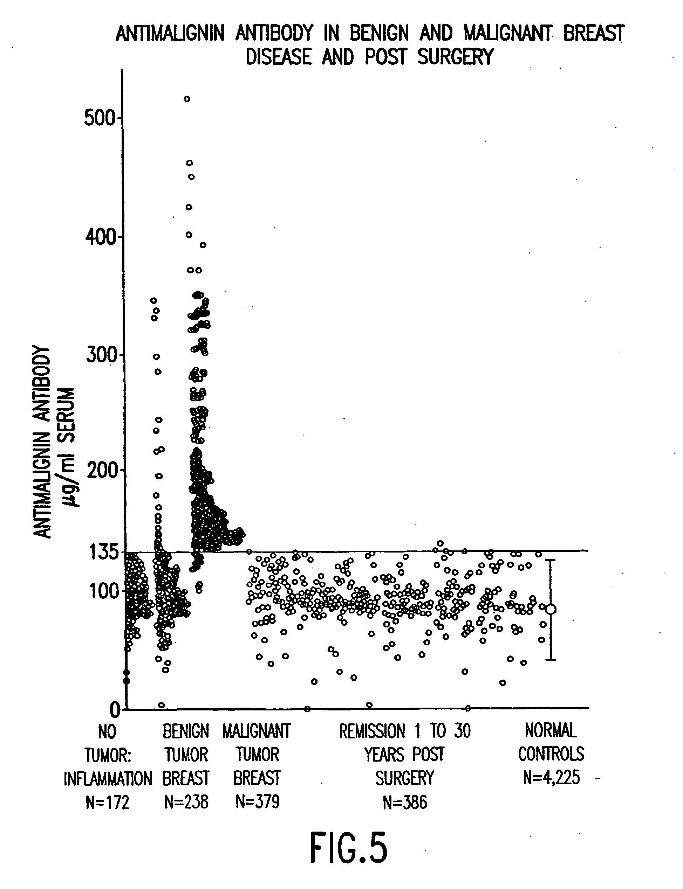

Figure 5 is a plot of the amount of antimalignin antibody present in the serum of patients with benign or malignant breast disease pre-and post surgery.

Figure 6 is a graph showing the concentration of Replikins observed in hemagglutinin of influenza B and influenza A strain, H1N1, on a year by year basis from 1918 through 2001.

Figure 7 is a graph of the Replikin concentration observed in hemagglutinin of influenza A strains, H2N2 and H3N2, as well as an emerging strain defined by its constituent Replikins, designated H3N2(R), on a year by year basis from 1950 to 2001.

Figure 8 is a graph depicting the Replikin count per year for several virus strains, including the coronavirus nucleocapsid Replikin, from 1917 to 2002.

Figure 9 is a chart depicting the mean Replikin count per year for nucleocapsid coronavirus isolates.

Figure 10 is a chart depicting the Replikin count per year for H5N1 Hemagglutinins.

Figure 11 is a graph illustrating a rapid increase in the concentration of Replikin patterns in the hemagglutinin protein of the H5N1 strain of influenza prior to the outbreak of three "Bird Flu" epidemics. Figure 11 illustrates that increasing Replikin concentration ('Replikin Count') of hemagglutinin protein of H5N1 preceded three "Bird Flu" Epidemics. In H5N1 influenza, the increasing strain-specific Replikin concentration (Replikin Count, Means+/-SD) 1995 to 1997 preceded the Hong Kong H5N1 epidemic of 1997 (E1); the increase from 1999 to 2001 preceded the epidemic of 2001 (E2); and the increase from 2002 to 2004 preceded the epidemic in 2004 (E3). The decline in 1999 occurred with the massive culling of poultry in response to the E1 epidemic in Hong Kong.

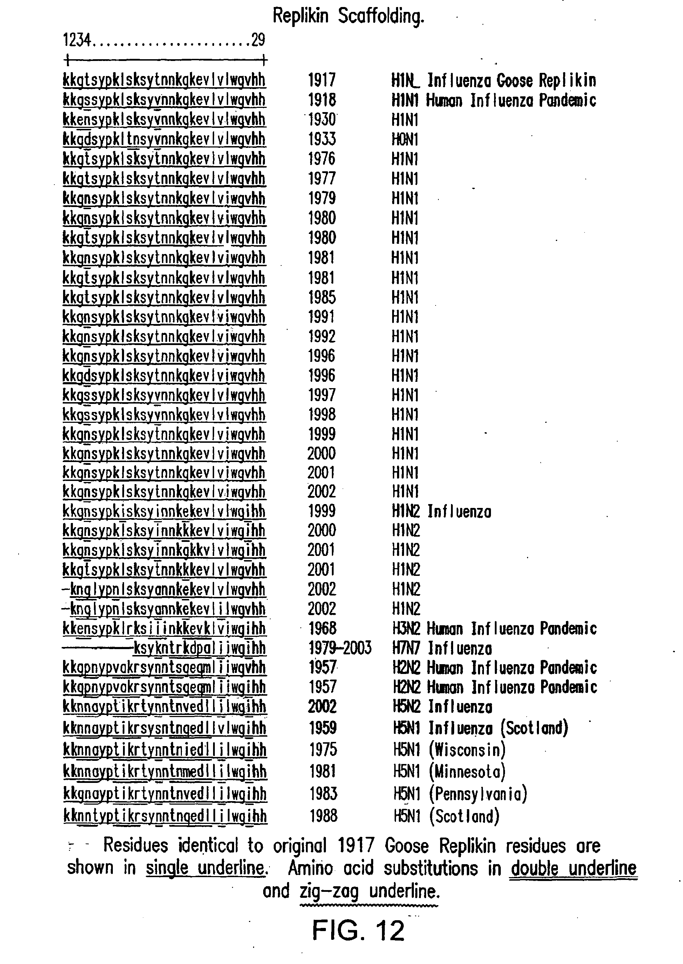

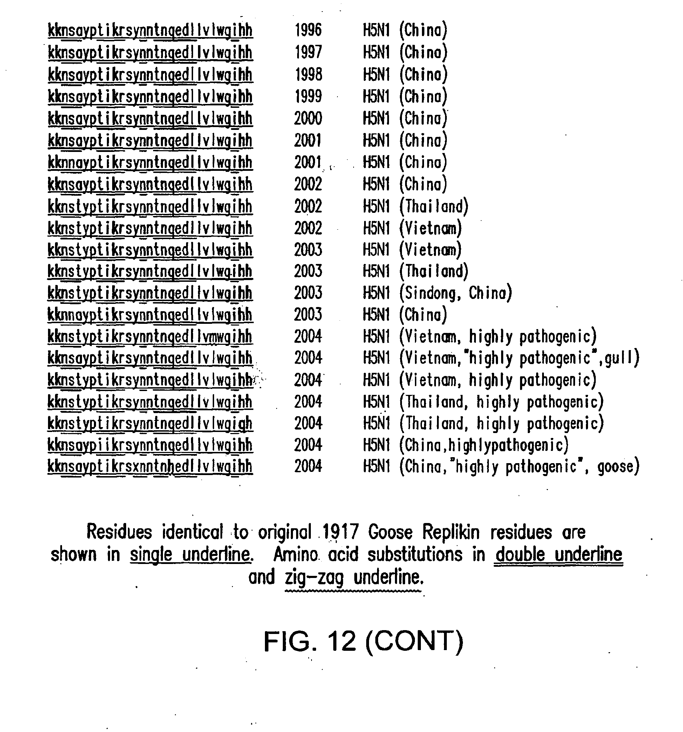

Figure 12 is a table illustrating Replikin Scaffolds occurring in substantially fixed amino acid positions in different proteins.

Figure 13 is a table providing Replikin sequences present in hemagglutinins of Influenza B viruses in each year for which amino acid sequences were available (1940-2001).

Figure 14 is a table providing H1N1 Replikin Sequences present in HINI hemagglutinins of influenza viruses in each year for which amino acid sequences were available (1918-2000).

Figure 15 is a table providing Replikin Sequences present in hemagglutinins of Influenza H2N2 viruses in years 1957-2000.

Figure 16 is a table providing H3N2 Replikin Sequences present in H3N2 hemagglutinins of Influenza viruses in each year for which amino acid sequences were available (1968-2000).

Figure 17 is a table providing the relationship of Replikin structure in influenza virus, SARS virus and other rapidly replicating viruses and malignancies to increased host mortality.

### DETAILED DESCRIPTION OF THE INVENTION

### Definitions

As used herein, a Replikin peptide or Replikin protein is an amino acid sequence comprising 7 to about 50 amino acids comprising: (1) at least one lysine residue located six to ten amino acid residues from a second lysine residue; (2) at least one histidine residue; (3) at least 6% lysine residues. Similarly, a Replikin sequence is the nucleic acid sequence encoding a Replikin peptide.

As used herein "Replikin Scaffold" refers to a series of conserved Replikin peptides wherein each of said Replikin peptide sequences comprises about 16 to about 30 amino acids and further comprises: (1) a terminal lysine and optionally a lysine immediately adjacent to the terminal lysine; (2) a terminal histidine and a histidine immediately adjacent to the terminal histidine; (3) a lysine residue 6 to 10 amino acid residues from another lysine residue; and (4) at least about 6% lysine. "Replikin Scaffold" also refers to an individual member or a plurality of members of a series of a "Replikin Scaffold."

As used herein, an "earlier-arising" virus or organism is a specimen of a virus or organism collected from a natural source of the virus or organism on a date prior to the date on which another specimen of the virus or organism was collected. A "later-arising" virus or organism is a specimen of a virus or organism collected from a natural source of the virus or organism on a date subsequent to the date on which another specimen of the virus or organism was collected.

As used herein, "emerging strain" refers to a strain of a virus identified as having an increased or increasing concentration of Replikin sequences in one or more of its protein sequences relative to the concentration of Replikins in earlier-arising isolates of such strain of virus or in other strains of such virus. The increased or increasing concentration of Replikins occurs over a period of at least about six months, and preferably over a period of at least about one year, most preferably over a period of at least about three years or more, for example, in influenza virus, but may be a much shorter period of time:

"Functional derivatives" of Replikins as described herein are fragments, variants, analogs, or chemical derivatives of the Replikins, which retain at least a portion of the immunological cross reactivity with an antibody specific for the Replikin. A fragment of the Replikin peptide refers to any subset of the molecule. Variant peptides may be made by direct chemical synthesis, for example, using methods well known in the art. An analog of a Replikin to a non-natural protein substantially similar to either the entire protein or a fragment thereof. Chemical derivatives of a Replikin contain additional chemical moieties.

As used herein, "homology," as percent of all amino acids within a given replikin, is critical for the amino acids which define the replikin structure, i.e.,lysines and histidine, at the positions maintained by these "defining" amino acids and less important or not important at all for the other amino acids within the replikin structure, as shown for HIV TAT protein in previous applications; conservation is defined in terms of these relatively invariant "defining" amino acids; specific variation or substitution in other than "defining" amino acids is the subject of this application, where these are seen to be non-random and associated as here shown for example with pandemics, are not as insignificant as previously thought; by repeating with given functions they are shown to represent sub-sets of the basic replikin scaffold.

As used herein, "mutation" refers to change in the structure and properties of an organism caused by substitution of amino acids. In contrast, the terms "conservation, "conserved" or related words, as used herein, refer to conservation of particular amino acids due to lack of substitution.

As used herein, the term "peptide" or "protein" refers to a compound of two or more amino acids in which the carboxyl group of one is united with an amino group of another, forming a peptide bond. The term peptide is also used to denote the amino acid sequence encoding such a compound.

As used herein, "isolated" or "synthesized" peptide or biologically active portion thereof refers to a peptide that is after purification substantially free of cellular material or other contaminating proteins or peptides from the cell or tissue source from which the peptide is derived, or substantially free from chemical precursors or other chemicals when chemically synthesized by any method, or substantially free from contaminating peptides when synthesized by recombinant gene techniques. Isolation may be accomplished *in vivo, in vitro,* and now, by proteomic software methods "*in silico*."

As used herein, "Replikin count" or "Replikin concentration" refers to the number of Replikins per 100 amino acids in a protein or organism. A higher Replikin count in a first strain of virus or organism has been found to correlate with more rapid replication of the first virus or organism as compared to a second, earlier- or later-arising strain of the virus or organism having a lower Replikin count.

### I. INCREASED REPLIKIN COUNT CORRELATED WITH INFLUENZA EPIDEMICS AND PANDEMICS

Our study concerning influenza virus protein sequences and influenza epidemiology over the past 100 years is providing methods of predicting increased replication and virulence in influenza. A review of historic and current data from influenza virus hemagglutinin protein sequences reveals a four to ten-fold increase in the concentration of strain-specific influenza Replikins in one of each of the four major strains of influenza, namely, influenza B, (A)HIN1, (A)H2N2 and (A)H3N2, in strains related to influenza epidemics from 1902 to 2001. Increases in concentration of Replikins in hemagglutinin protein related to epidemics are further found to be due to the reappearance of at least one specific Replikin composition from 1 to up to 64 years after its disappearance, plus the emergence of new strain-specific Replikin compositions.

Prior to the discovery of Replikins in the influenza genome, only serological hemagglutinin and antibody classification had previously been described in influenza. No strain-specific conserved peptide sequences had been described. Further, no changes in concentration and composition of any strain-specific peptide sequences had been described that correlated with epidemiologically documented epidemics or rapid replication. As such, no strain-specific chemical structures were known with which to predict the strains that would predominate in coming influenza seasons, nor to devise annual mixtures of whole-virus strains for vaccines.

The high degree of conservation of Replikin structures observed, whereby the identical structure can persist for 100 years, or reappear after an absence of from one to 64 years, however, indicates that what was previously thought to be change due to random substitution of amino acids in influenza proteins is more likely to be change due to an organized process of conservation of Replikins.

In view of the historical data, the monitoring of increases in Replikin concentration in influenza provided for a tool for predicting future epidemics. The tool has effectively predicted a number of recent influenza outbreaks. For example, a recent sharp increase in H3N2 Replikin concentration (1997 to 2000), the largest in H3N2's history, and the reappearance of specific Replikin compositions that were last seen in the high mortality H3N2 pandemic of 1968, and in the two high mortality epidemics of 1975 and 1977, but were absent for 20-25 years, together predicted the H3N2 epidemic of 2002.

### Pandemics in Influenza B, H1N1, H2N2 and H3N3

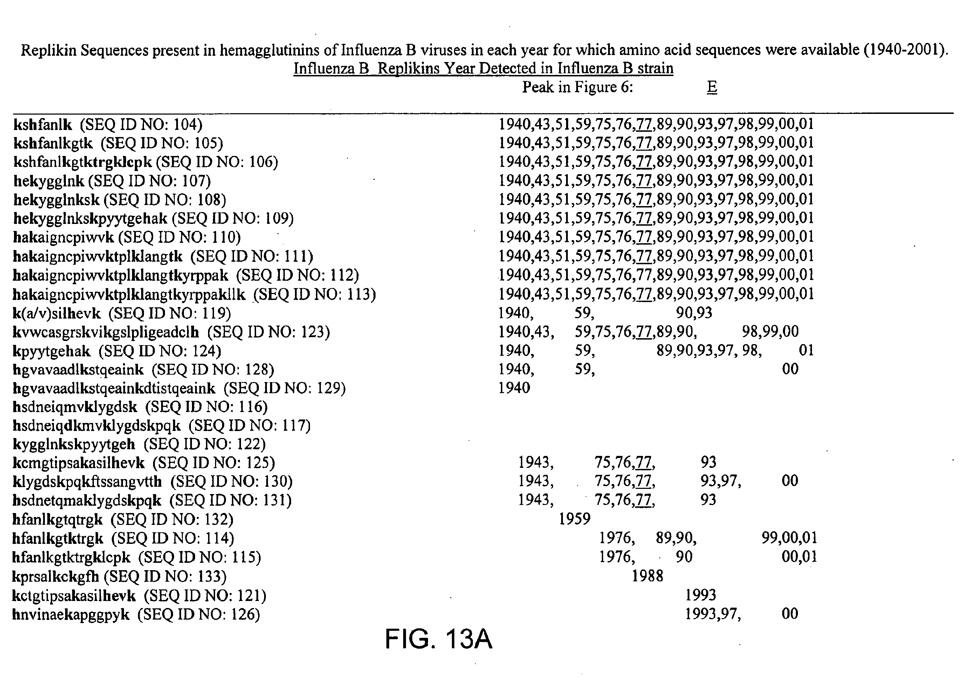

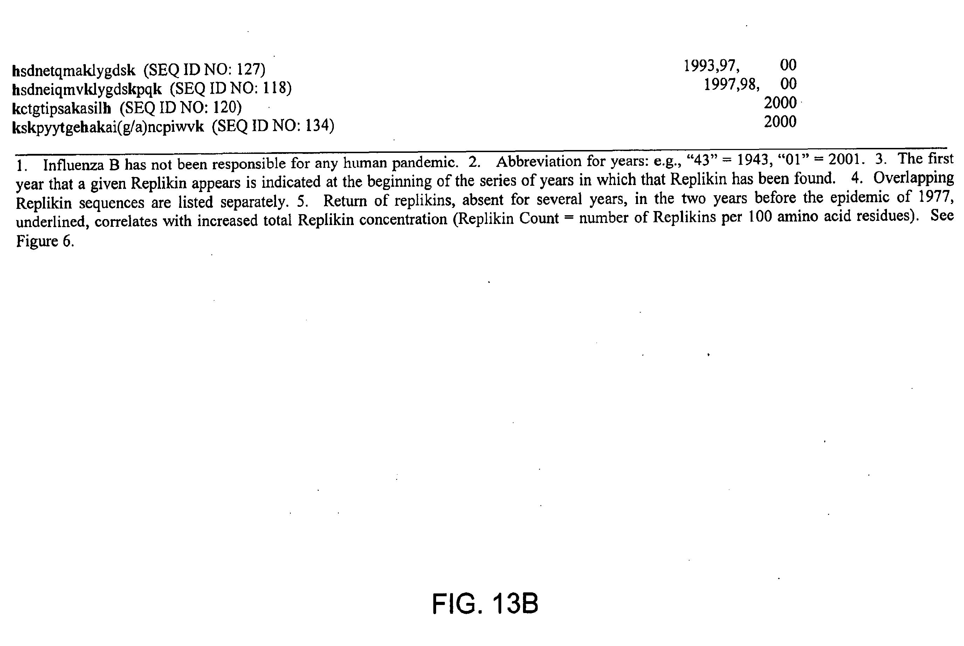

Of particular interest, we have observed that at least one Replikin per 100 amino acids is present in the hemagglutinin proteins of almost all of the individual strains of influenza viruses examined. The Replikin sequences that have been observed to occur in the hemagglutinin proteins of isolates of each of the four prevalent strains of influenza virus, influenza B, H1N1, H2N2, and H3N2, for each year that amino acid sequence data are available (1902-2001), are shown in Figures 13, 14, 15 and 16.

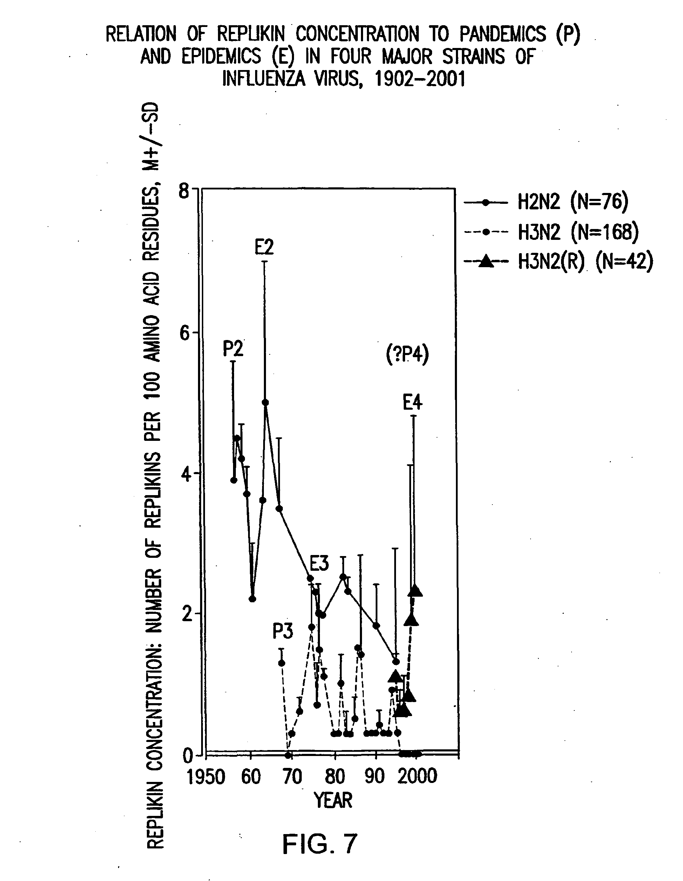

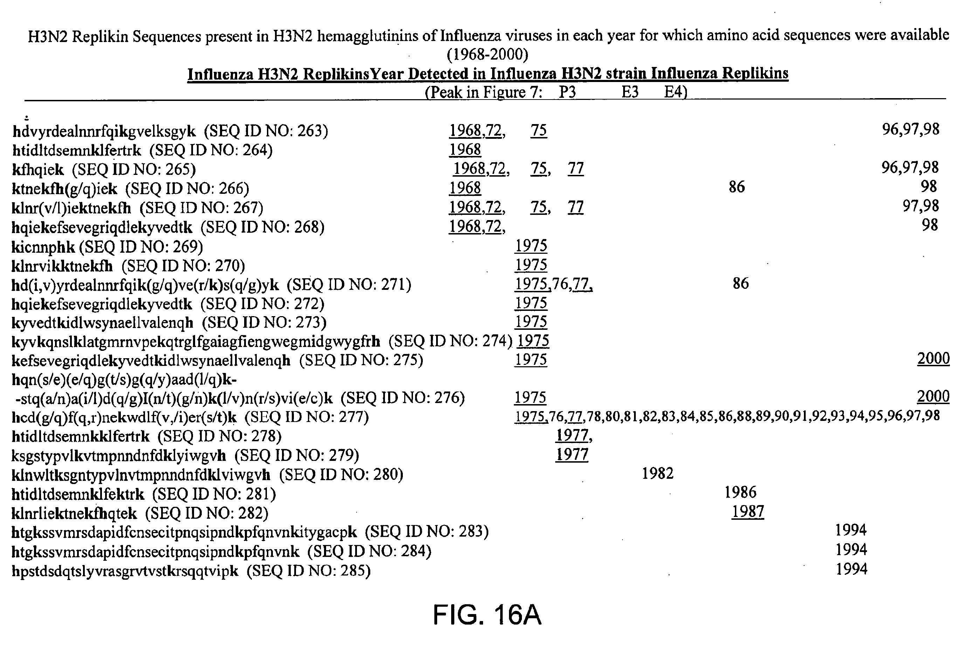

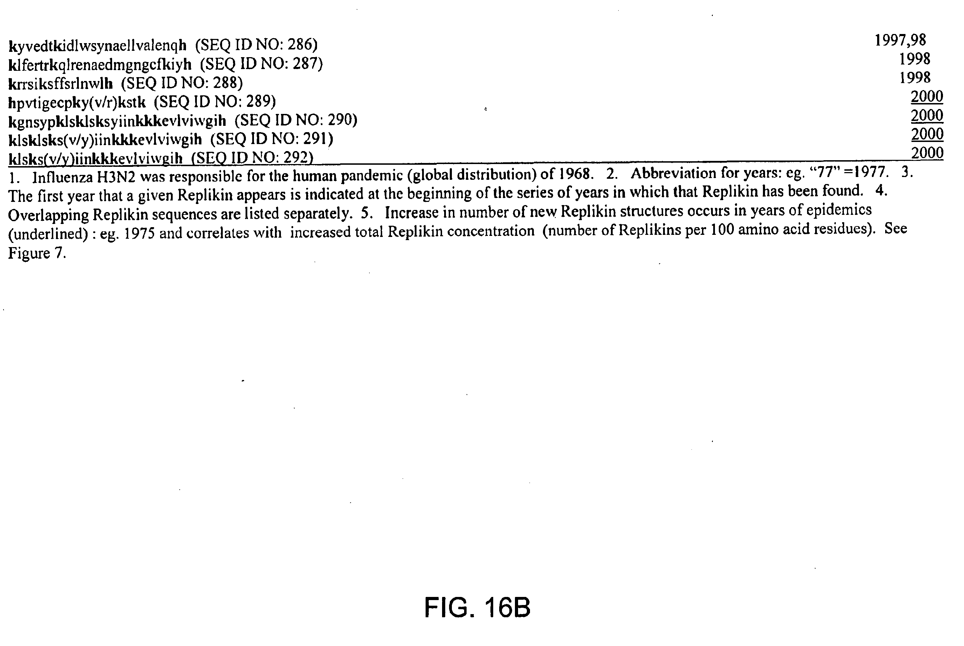

Each influenza A strain has been responsible for one pandemic: in 1918, 1957, and 1968, respectively. The data in Figures 6 and 7 show that at least one Replikin per 100 amino acids is present in each of the influenza hemagglutinin proteins of all isolates of the four common influenza viruses examined, suggesting a function for Replikins in the maintenance of survival levels of replication. In the 1990s, during the decline of the H3N2 strain, there were no Replikins in many isolates of H3N2, but a high concentration of new Replikins appeared in H3N2 isolates, which define the emergence of the H3N2(R) strain. See Figure 16.

Several properties of Replikin concentration are seen in Figure 6 and Figure 7 to be common to all four influenza virus strains. First, the concentration is cyclic over the years, with a single cycle of rise and fall occurring over a period of two to thirty years. This rise and fall is consistent with the known waxing and waning of individual influenza virus strain predominance by hemagglutinin and neuraminidase classification.

Second, peak Replikin concentrations of each influenza virus strain previously shown to be responsible for a pandemic relate specifically and individually to each of the three years of the pandemics. For example, for the pandemic of 1918, where the influenza virus strain, H1N1, was shown to be responsible, a peak concentration of the Replikins in H1N1 independently occurred (P1); for the pandemic of 1957, where H2N2 emerged and was shown to be responsible, a peak concentration of the Replikins in H2N2 occurred (P2); and for the pandemic of 1968, where H3N2 emerged and was shown to be the cause of the pandemic, a peak concentration of the Replikins in H3N2 occurred (P3).

Third, in the years immediately following each of the above three pandemics, the specific Replikin concentration decreased markedly, perhaps reflecting the broadly distributed immunity generated in each case. Thus, this post-pandemic decline is specific for H1N1 immediately following the pandemic (P1) for which it was responsible, and is not a general property of all strains at the time. An increase of Replikin concentration in influenza B repeatedly occurred simultaneously with the decrease in Replikin concentration in H1N1, e.g., EB1 in 1951 and EB2 in 1976, both associated with influenza B epidemics having the highest mortality. (Stuart-Harris, et al., Edward Arnold Ltd. (1985).

Fourth, a secondary peak concentration, which exceeded the primary peak increase in concentration, occurred 15 years after each of the three pandemics, and this secondary peak was accompanied by an epidemic: 15 years after the 1918 pandemic in an H1N1 'epidemic' year (E1); eight years after the 1957 pandemic in an H2N2 'epidemic' year (E2); and seven years after the 1968 pandemic in an H3N2 'epidemic' year (E3). These secondary peak concentrations of specific Replikins may reflect recovery of the strain.

Fifth, peaks of each strain's specific Replikin concentration frequently appear to be associated with declines in Replikin concentration of one or both other strains, suggesting competition between strains for host sites. Sixth, there is an apparent overall tendency for the Replikin concentration of each strain to decline over a period of 35 years (H2N2) to 60 years (influenza B). This decline cannot be ascribed to the influence of vaccines because it was evident in the case of influenza B from 1940 to 1964, prior to common use of influenza vaccines. In the case of influenza B. Replikin recovery from the decline is seen to occur after 1965, but Replikin concentration declined again between 1997 and 2000 (Figure 6). This correlates with the low occurrence of influenza B in recent case isolates. H1N1 Replikin concentration peaked in 1978-1979 (Figure 6) together with the reappearance and prevalence of the H1N1 strain, and then peaked in 1996 coincident with an H1N1 epidemic. (Figure 6). H1N1 Replikin concentration also declined between 1997 and 2000, and the presence of H1N1 strains decreased in isolates obtained during these years. For H2N2 Replikins, recovery from a 35 year decline has not occurred (Figure 7), and this correlates with the absence of H2N2 from recent isolates. For H3N2, the Replikin concentration of many isolates fell to zero during the period from 1996 to 2000, but other H3N2 isolates showed a significant, sharp increase in Replikin concentration. This indicates the emergence of a substrain of H3N2, which is designated herein as H3N2(R).

As discussed above, Figures 6 and 7 demonstrate that frequently, a one to three year stepwise increase is observed before Replikin concentration reaches a peak. This stepwise increase precedes the occurrence of an epidemic, which occurs concurrently with the Replikin peak. Thus, the stepwise increase in concentration of a particular strain is a signal that particular strain is the most likely candidate to cause an epidemic or pandemic.

Replikin concentration in the H3N2(R) strain of influenza virus was observed to increase between 1997 and 2000 (Figure 7). The resulting epidemic in 2002 demonstrated the predictive value of increases in Replikin concentration prior to an epidemic. Three similar previous peak increases in H3N2 Replikin concentration were seen to have occurred in the H3N2-based pandemic of 1968 (Figure 7), when the strain first emerged, and in the H3N2-based epidemics of 1972 and 1975 (Figure 7). Each of these pandemic and epidemics was associated with excess mortality. (Ailing, et al., Am J. Epidemiol.,113(1):30-43 (1981).

The rapid ascent in concentration of the H3N2(R) subspecies of the H3N2 Replikins in 1997-2000, therefore, statistically represented an early warning of an approaching severe epidemic or pandemic. The prediction was demonstrated correct in that a H3N2 epidemic occurred in Russia in 2000 (Figure 7, E4); and a CDC report of December 2001 stating that, in 2001, H3N2 was the most frequently isolated strain of influenza virus worldwide. (Morbidity and Mortality Weekly Reports (MMWR), Center for Disease Control; 50(48):1084-68 (Dec.7, 2001).

### Composition of Replikins in Strains of Influenza Virus B

Of a total of 26 Replikins identified in the influenza virus B strain (Figure 13), the following ten Replikins are present in every influenza B isolate examined from 1940-2001. Overlapping Replikin sequences are listed separately.
KSHFANLK (SEQ ID NO:__)
KSHFANLKGTK (SEQ ID NO: __)
KSHFANLKGTKTRGKLCPK (SEQ ID NO: __)
HEKYGGLNK (SEQ ID NO: __)
HEKYGGLNKSK (SEQ ID NO: __)
HEKYGGLNKSKPYYTGEHAK (SEQ ID NO: __)
HAKAIGNCPIWVK (SEQ ID NO: __)
HAKAIGNCPIWVVKKTPLKLANGTK (SEQ ID NO: __)
HAKAIGNCPIWVKTPLKLANGTKYRPPAK (SEQ ID NO: __)
HAKAIGNCPIWVKTPLKLANGTKYRPPAKLLK (SEQ ID NO: __)

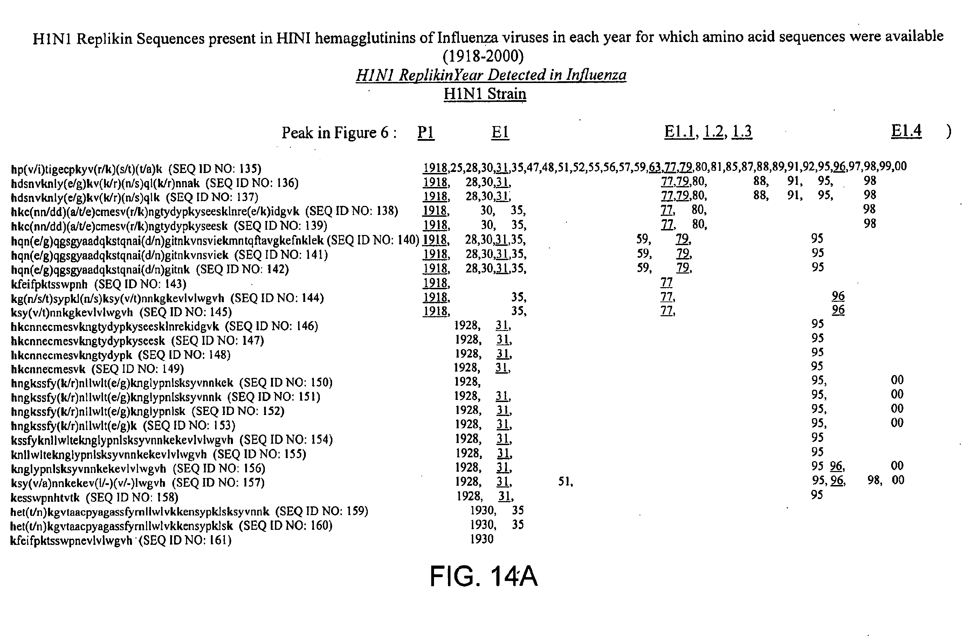

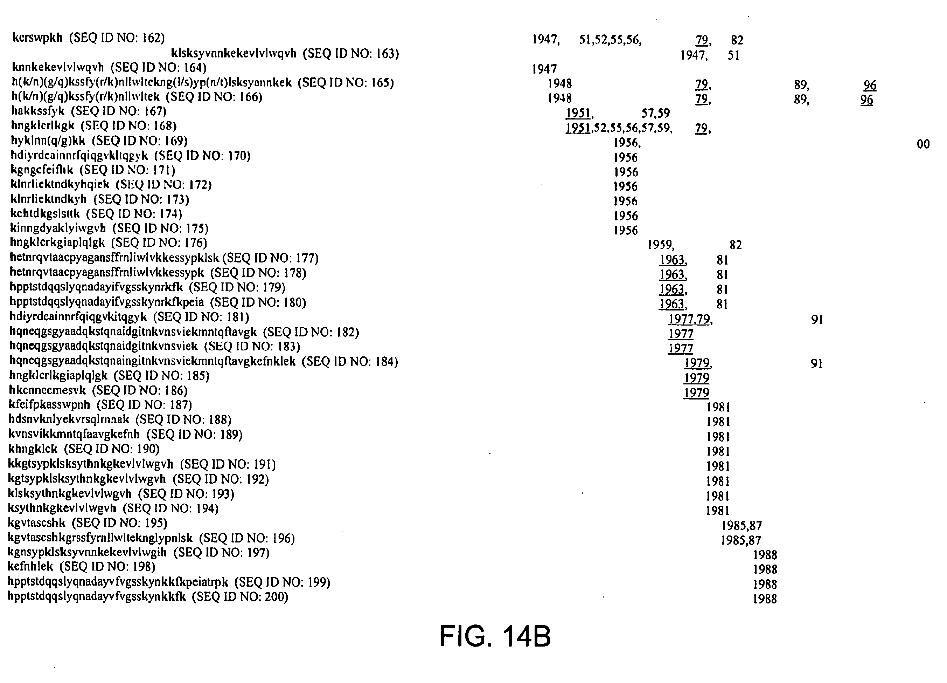

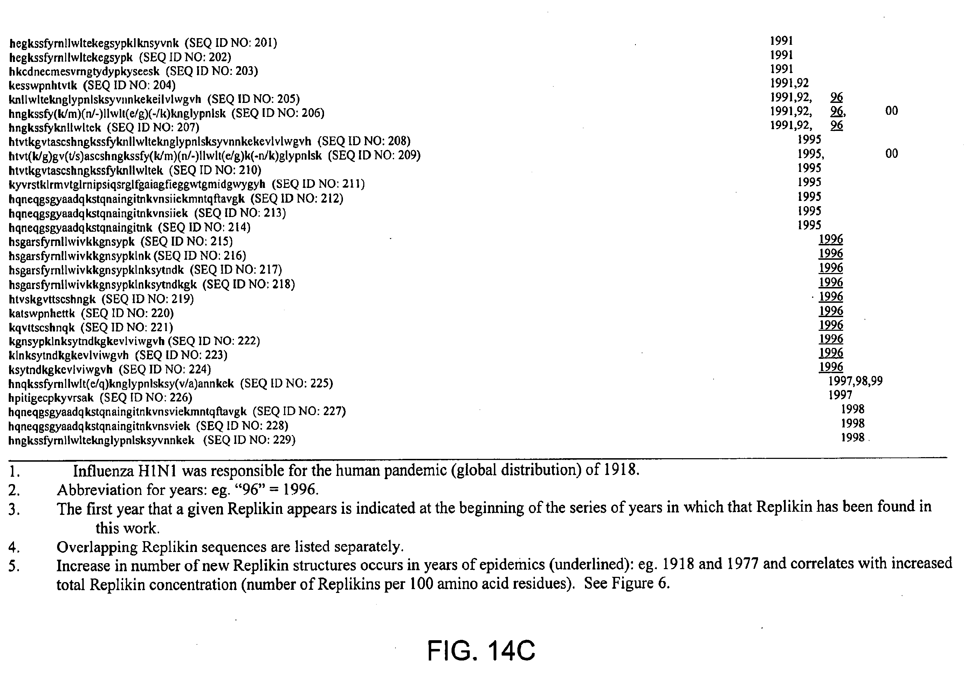

Figures 13 and 14 indicate that there appears to be much greater stability of the Replikin structures in influenza B hemagglutinins compared with H1N1 Replikins. Influenza B has not been responsible for any pandemic, and it appears not to have an animal or avian reservoirs. (Stuart-Harris et al., Edward Arnold Ltd., London (1985)).

### Genetic Drift in Strains of Influenza

In each case of influenza pandemic or epidemic, new Replikins emerge. There has been no observation of two of the same Replikins in a given hemagglutinin in a given isolate. To what degree the emergence of a new Replikin represents mutations versus transfer from another animal or avian pool is unknown. In some cases each year, one or more of the original Replikin structures is conserved, while at the same time, new Replikins emerge. For example, in influenza virus B hemagglutinin, five Replikins were constantly conserved between 1919 and 2001, whereas 26 Replikins came and went during the same period (some recurred after a several year absence). The disappearance and re-emergence years later of a particular Replikin structure suggests that the Replikins return from another virus host pool rather than through de novo mutation.

It has been believed that changes in the activity of different influenza strains are related to sequence changes in influenza hemagglutinins, which in turn are the products of substitutions effected by one of two poorly understood processes: i) antigenic drift, thought to be due to the accumulation of a series of point mutations in the hemagglutinin molecule, or ii) antigenic shift, in which the changes are so great that genetic reassortment is postulated to occur between the viruses of human and non-human hosts. First, the present data suggests that the change in activity of different influenza strains, rather than being related to non-specific sequence changes, are based upon, or relate to, the increased concentration of strain-specific Replikins and strain-specific increases in the replication associated with epidemics.

In addition, the data were examined for a possible insight into which sequence changes are due to "drift" or "shift," and which are due to conservation, storage in reservoirs, and reappearance. The data suggest that the epidemic-related increase in Replikin concentration is not due to the duplication of existing Replikins per hemagglutinin, but is due to the reappearance of at least one Replikin composition from 1 to up to 59 years after its disappearance, plus in the A strains only, the emergence of new strain-specific Replikin compositions (Figures 13-16). Thus the increase in Replikin concentration in the influenza B epidemics of 1951 and 1977 are not associated with the emergence of new Replikin compositions in the year of the epidemic but only with the reappearance of Replikin compositions which had appeared in previous years then disappeared (Figure 13).

In contrast, for the A strains, in addition to the reappearance of previously disappeared virus Replikins, new compositions appear (e.g., in H1N1 in the year of the epidemic of 1996, in addition to the reappearance of 6 earlier Replikins, 10 new compositions emerged). Since the A strains only, not influenza B, have access to non-human animal and avian reservoirs, totally new compositions probably derive from non-human host reservoirs rather than from mutations of existing human Replikins which appear to bear no resemblance to the new compositions other than the basic requirements of the "3-point recognition" of the Replikin algorithm (Figures 13-17). The more prolific nature of H1N1 compared with B, and the fact that pandemics have been produced by the three A strains only, but not by the B strain, both may also be a function of the ability of the human A strains to receive new Replikin compositions from non-human viral reservoirs.

Some Replikins have appeared in only one year, disappeared, and not reappeared to date (Figures 13-16). Other Replikins disappear from one to up to 81 years, when the identical Replikin sequence reappears. Key Replikin 'k' and 'h' amino acids, and the spaces between them, are conserved during the constant presence of particular Replikins over many years, as shown in Figures 13-16 for the following strain-specific Replikins: ten of influenza B, the single Replikin of H1N1, and the single Replikin of H3N2 as well as for the reappearance of identical Replikins after an absence.

Despite the marked replacement or substitution activity of other amino acids both inside the Replikin structure and outside it in the rest of the hemagglutinin sequences, influenza Replikin histidine (h) appears never to be, and lysine (k) is rarely replaced. Examples of this conservation are seen in the H1N1 Replikin "hp(v/i)tigecpkyv(r/k)(s/t)(t/a)k" (SEQ ID NO: __), constant between 1918 and 2000, in the H3N2 Replikin "hcd(g/q)f(q,r)nekwdlf(v/i)er(s/t)k" (SEQ ID NO: __), constant between 1975 and 1998 and in the H3N2 Replikin "hqn(s/e)(e/q)g(t/s)g(q/y)aad(l/q)kstq(a/n)a(i/l)d(q/g)l(n/t)(g/n)k,(l/v)n(r/s) vi(e/c)k" (SEQ ID NO: __) which first appeared in 1975, disappeared for 25 years, and then reappeared in 2000. While many amino acids were substituted, the basic Replikin structure of two Lysines, six to ten residues apart, one histidine, a minimum of 6% lysine in not more than approximately 50 amino acids, was conserved.

Totally random substitution would not permit the persistence of these H1N1 and H3N2 Replikins, nor from 1902 to 2001 in influenza B the persistence of 10 Replikin structures, nor the reappearance in 1993 of a 1919 18-mer Replikin after an absence of 74 years. Rather than a random type of substitution, the constancy suggests an orderly controlled process, or in the least, protection of the key Replikin residues so that they are fixed or bound in some way: lysines, perhaps bound to nucleic acids, and histidines, perhaps bound to respiratory redox enzymes. The mechanisms, which control this conservation, are at present unknown.

### II. CONSERVATION AND GENETIC DRIFT PROVIDE EARLY WARNING MECHANISM FOR VACCINE DEVELOPMENT

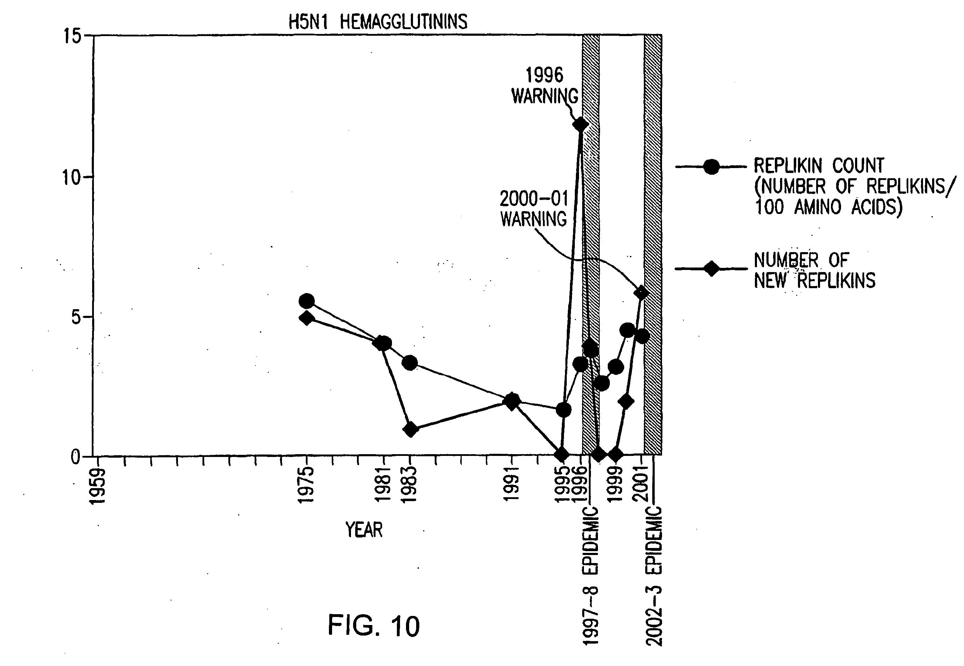

In the case of H1N1 Replikins, the two Replikins present in the P1 peak associated with the 1918 pandemic were not present in the recovery E1 peak of 1933, which contains 12 new Replikins. Constantly conserved Replikins, therefore, are the best choice for vaccines, either alone or in combination. However, even recently appearing Replikins accompanying one year's increase in concentration frequently persist and increase further for an additional one or more years, culminating in a concentration peak and an epidemic, thus providing both an early warning and time to vaccinate with synthetic Replikins (see for example, H1N1 in the early 1990's, Figure 6; see also, for example, H5N1 1995-2002, Figure 10 and Figure 11).

The data in Figures 7, 8, 10 and 11 demonstrate a direct relationship between the presence and concentration Replikins in influenza protein sequences and the occurrence of pandemics and epidemics of influenza. Thus, analysis of the influenza virus hemagglutinin protein sequence for the presence and concentration of Replikins provides a predictor of influenza pandemics and/or epidemics, as well as a target for influenza vaccine formulation. It is worth noting again with reference to this data, previously, no strain-specific chemical structures were known with which to predict the strains that would predominate in coming influenza seasons, nor to devise annual mixtures of whole-virus strains for vaccines.

Similar to the findings of strain-specific Replikin Count increases in the influenza group one to three years prior to the occurrence of a strain-specific epidemics, the increase in Replikin Count of the coronavirus nucleocapsid protein has also been identified. Replikin Counts of the coronavirus nucleocapsid protein has increased as follows: 3.1 (±1.8) in 1999; 3.9(±1.2) in 2000; 3.9 (±1.3) in 2001; and 5.1 (±3.6) in 2002. This pre-pandemic increase supports the finding that a coronavirus was responsible for the 2003 SARS pandemic. (See Figure 8 and Table 3)

Thus, monitoring Replikin structure and Replikin Count provides a means for developing synthetic strain-specific preventive vaccination and antibody therapies against the 1917-1918 Goose Replikin and its modified and accompanying Replikins as observed in both influenza and coronavirus strains.

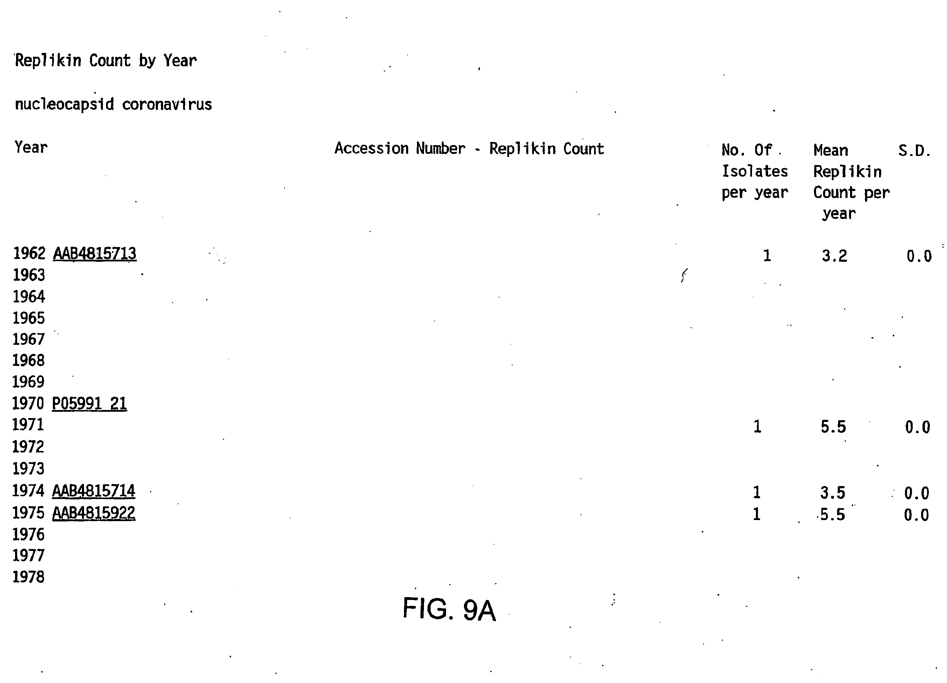

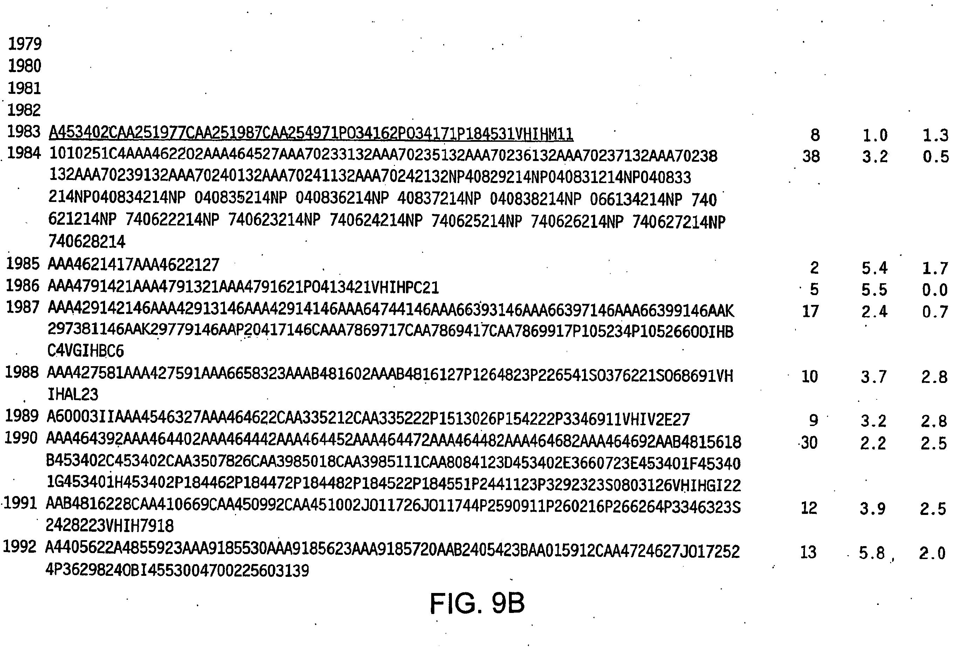

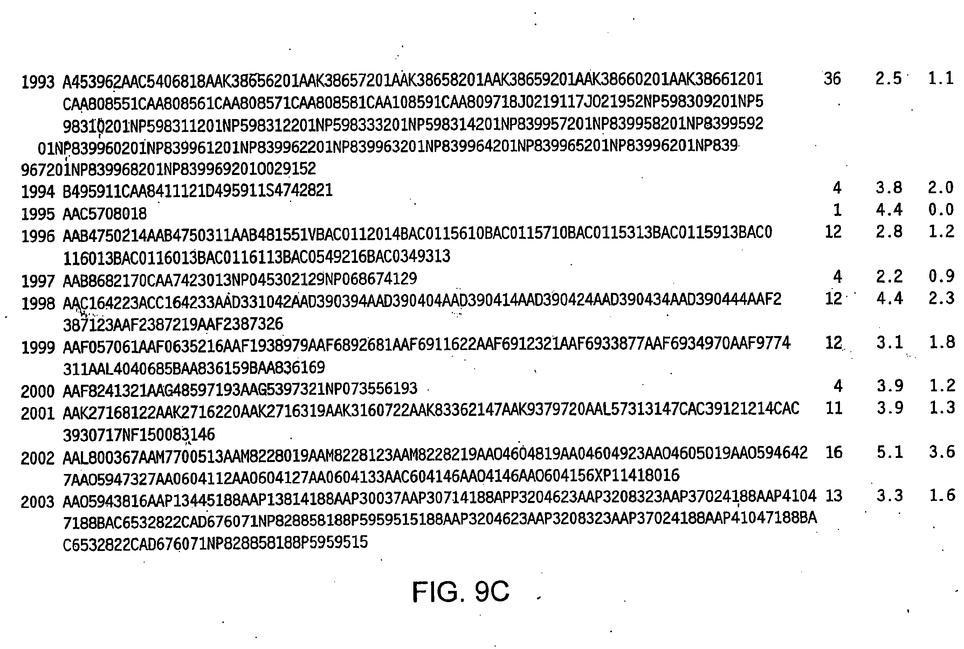

Figure 9 depicts the automated Replikin analysis of nucleocapsid coronavirus proteins for which the protein sequence is available on isolates collected from 1962 to 2003. Each individual protein is represented by an accession number and is analyzed for the presence of Replikins. The Replikin Count (number of Replikins per 100 amino acid) is automatically calculated as part of the automated Replikin analysis. For each year, the mean (± Standard deviation (S.D.)) Replikin Count per year is automatically calculated for all Replikin Counts that year. This example of early warning of increasing replication, before an epidemic, of a particular protein (the nucleocapsid protein) in a particular virus strain (the coronavirus) is comparable to the increase seen in strains of influenza virus preceding influenza epidemics and pandemics (Figures 6, 7, 10 and 11). It may be seen that the Replikin Count rose from 1999 to 2002, consistent with the SARS coronavirus pandemic, which emerged at the end of 2002 and persisted into 2003. Figure 8 provides a graph of the Replikin Counts for several virus strains, including the coronavirus nucleocapsid Replikin, from 1917 to 2002.

### III. REPLIKINS IN INFLUENZA

Only one Replikin "hp(v/i)tigecpkyv-(r/k)(s/t)(t/a)k" is present in every H1N1 isolate for which sequences are available from 1918, when the strain first appeared and caused the pandemic of that year, through 2000. (Figure 14) ("(v/i)" indicates that the amino acid v or i is present in the same position in different years.) Although H1N1 contains only one persistent Replikin, H1N1 appears to be more prolific than influenza B. There are 95 different Replikin structures in 82 years on H1N1 versus only 31 different Replikins in 62 years of influenza B isolates (Figures 13 and 14). An increase in the number of new Replikin structures occurs in years of epidemics (Figures 13-16) and correlates with increased total Replikin concentration (Figures 6, 7, 10 and 11).

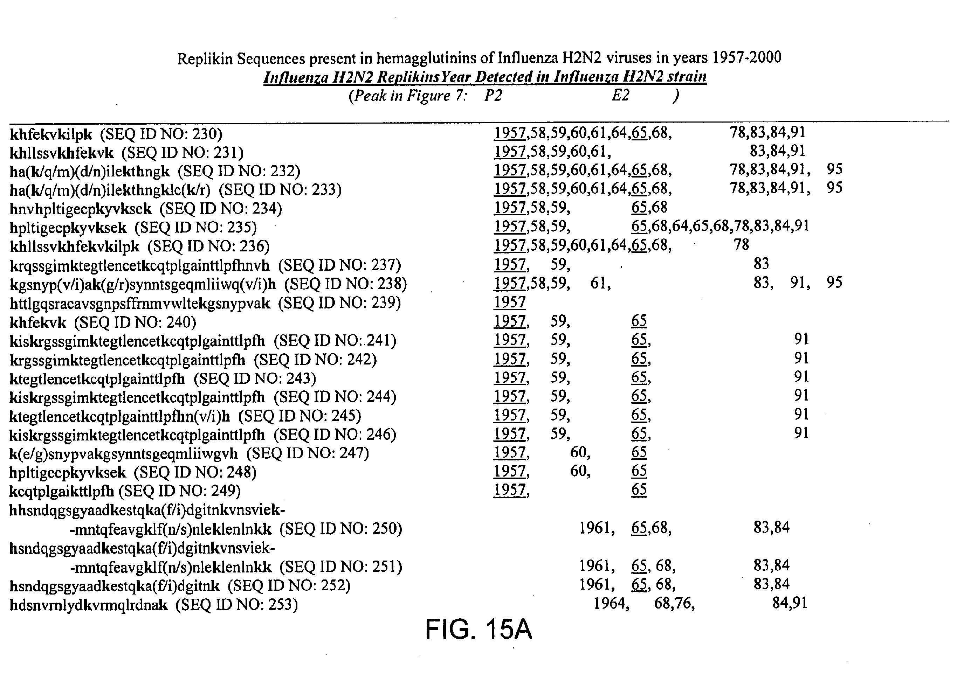

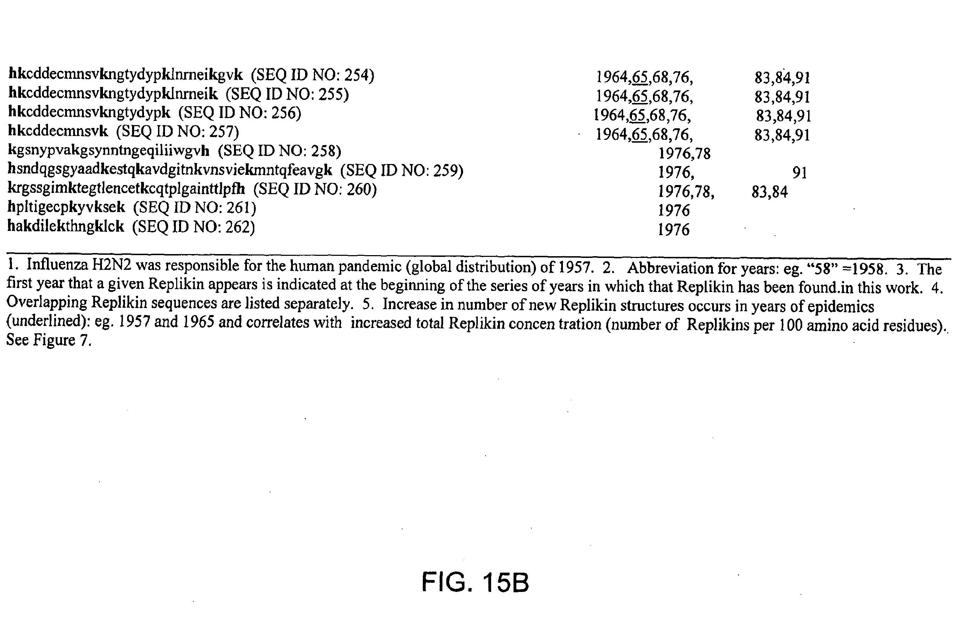

Influenza H2N2 Replikins: Influenza H2N2 was responsible for the human pandemic of 1957. Three of the 20 Replikins identified in that strain for 1957 were conserved in each of the H2N2 isolates available for examination on PubMed until 1995 (Figure 15).
ha(k/q/m)(d/n)ilekthngk (SEQ ID NO: __)
ha(k/q/m)(d/n)ilekthngklc(k/r) (SEQ ID NO: __)
kgsnyp(v/i)ak(g/r)synntsgeqmliiwq(v/i)h (SEQ ID NO: __)

However, in contrast to H1N1, only 13 additional Replikins have been found in H2N2 beginning in 1961. This paucity of appearance of new Replikins correlates with the decline in the concentration of the H2N2 Replikins and the appearance of H2N2 in isolates over the years. (Figure 7).

Influenza H3N2 was responsible for the human pandemic of 1968. Five Replikins which appeared in 1968 disappeared after 1977, but reappeared in the 1990s (Figure 16). The only Replikin structure which persisted for 22 years was hcd(g/q)f(q/r)nekwdlf(v/i)er(s/t)k, which appeared first in 1977 and persisted through 1998. The emergence of twelve new H3N2 Replikins in the mid 1990s (Figure 16) correlates with the increase in Replikin concentration at the same time (Figure 7), and with the prevalence of the H3N2 strain in recent isolates together with the concurrent disappearance of all Replikins from some of these isolates (Figure 7), this suggests the emergence of the new substrain H3N2(R). The epidemic in November and December of 2003 of a new strain of H3N2 (Fujian) confirms this prediction made first in U.S. Provisional Appln. Ser. No. 60/303,396, filed July 9, 2001.

Figures 6, 7, 10 and 11 show that influenza epidemics and pandemics correlate with the increased concentration of Replikins in influenza virus, which is due to the reappearance of at least one Replikin from one to 59 years after its disappearance. Also, in the A strain only, there is an emergence of new strain-specific Replikin compositions (Figures 14-16, see also increase in number of new Replikins, pre-epidemic for H5N1 in Figures 6 and 7). Increase in Replikin concentration by repetition of individual Replikins within a single protein appears not to occur in influenza virus, but is seen in other organisms.

### IV. GOOSE REPLIKIN SCAFFOLD

We found that the Replikin in the hemagglutinin of an influenza virus isolated from a goose in 1917 (which we named the Goose Replikin) appeared in the next year in the H1N1 strain of influenza responsible for the 1918 pandemic, with only two substitutions as follows: kkg(t/s)sypklsksy(t/v)nnkgkevlvlwgvhh. Table 1 shows that the influenza 1917 Goose Replikin (GR) then was essentially conserved for 85 years, despite multiple minor substitutions and apparent translocations to other influenza strains. We have found that the 1917 influenza GR demonstrated apparent mobility between several influenza strains, appearing in H1N1 (the pandemic of 1918), in H2N2 (pandemic of 1957-58), in H3N2 (pandemic of 1968, epidemic in China and Russia 2000, Fujian strain epidemic 2003) and in H5N1 (epidemic in China 1997). In 1997 its structure was restored in H1N2 exactly to its 1918 structure KKGSSYPKLSKSYVNNKGKEVLVLWGVHH.

Replikin sequences that are correlatable with increased virulence and host mortality provide targets for predicting, identifying and treating emerging strains of influenza virus. Table I shows the Goose Replikin. The Goose Replikin and its homologues over the following nine decades of data on influenza strains have been shown to be a useful predictor of virulence in emerging strains of influenza virus. Taken together as a series, the Goose Replikin and its homologues fit the algorithm for a Replikin Scaffold, which requires a series of conserved Replikin peptides wherein each of said Replikin peptide sequences comprises about 16 to about 30 amino acids and further comprises: (1) a terminal lysine and optionally a lysine immediately adjacent to the terminal lysine; (2) a terminal histidine and a histidine immediately adjacent to the terminal histidine; (3) a lysine residue 6 to 10 amino acid residues from another lysine residue; and (4) at least about 6% lysine.

### V. REPLIKIN SCAFFOLD SEQUENCES CORRELATE WITH INCREASED VIRULENCE IN INFLUENZA

Replikin Scaffold sequences in influenza isolates that have a substitution in an amino acid identical to a substitution in one or more Replikin Scaffold sequences from influenza isolates that have demonstrated increased virulence or host mortality are useful as targets and therapies in emerging strains of influenza virus. The present invention therefore provides an isolated Replikin Scaffold peptide from a first strain of influenza virus comprising 16 to about 30 amino acids and further comprising
(1) a terminal lysine and optionally a lysine immediately adjacent to the terminal lysine;
(2) a terminal histidine and a histidine immediately adjacent to the terminal histidine,
(3) a lysine 6 to about 10 amino acids from another lysine; and
(4) at least 6% lysines
wherein the isolated Replikin Scaffold sequence of the first strain of influenza virus contains an amino acid substitution as compared to a Replikin Scaffold sequence of a second strain of influenza virus wherein the amino acid substitution is not the substitution of a histidine and wherein the Replikin Scaffold sequence of the second strain of influenza virus comprises
(1) 16 to about 30 amino acids;
(2) a terminal lysine and optionally a lysine immediately adjacent to the terminal lysine,
(3) a terminal histidine and a histidine immediately adjacent to the terminal histidine;
(4) a lysine 6 to about 10 amino acids from another lysine; and
(5) at least 6% lysines, and
wherein the substitution is additionally present in a Replikin Scaffold sequence of a third strain of influenza virus comprising
(1) 16 to about 30 amino acids;
(2) a terminal lysine and optionally a lysine immediately adjacent to the terminal lysine;
(3) a terminal histidine and a histidine immediately adjacent to the terminal histidine;
(4) a lysine 6 to about 10 amino acids from another lysine; and
(5) at least 6% lysines; and
wherein the presence of the substitution in the third strain of influenza virus is correlated with an increase in replication, virulence or host mortality as compared to the second strain of influenza virus.

In a preferred embodiment, the amino acid residue in the Replikin sequence of the second strain of influenza virus that is substituted with a different amino acid residue in the Replikin sequence of the first strain of influenza virus is located five amino acid residues from the terminal histidine.

In another preferred embodiment, the amino acid residue in the Replikin sequence of the second strain of influenza virus is substituted with an amino acid residue other than leucine in the isolated Replikin sequence of the first strain of influenza virus. In another preferred embodiment, the amino acid is substituted with any hydrophobic amino acid other than leucine. In another preferred embodiment, the amino acid is substituted with a methionine or an isoleucine. In another preferred embodiment, the isolated Replikin sequence is isolated from an H5N1 influenza virus.

The inventors have established using 100 years of epidemiological data that the concentration of Replikins in an influenza virus correlates with virulence of the virus. Using the same epidemiological data, the structure of particular highly conserved Replikin sequences has also been correlated with virulence and epidemics. A review of individual sequence changes in highly conserved Replikin sequences over time within the same 100 years of epidemiological data has demonstrated retrospective and prospective predictive capacity.

Replikin Scaffolds in the Goose Replikin Scaffold have likewise been demonstrated to provide targets for treating emerging strains of influenza virus containing Goose Replikin homologues. For example, a 29 amino acid Replikin Scaffold peptide of a highly pathogenic 2004 strain of H5N1 in Vietnam (labeled "2004 H5N1 Vietnam, highly pathogenic" in Table 1) complemented with a short synthetic Replikin sequence known as a UTOPE and a Keyhole Limpet Hemocyanin adjuvant provided a strong immune response in rabbits and chickens when injected subcutaneously. See Example 7.

Table 1 provides the Goose Replikin and its homologues up to 2006. The scaffolding in Table 1 demonstrates that constant length, constant lysines at the amino terminal, and constant histidine residues at the carboxy terminal are conserved in different strains in a fixed scaffold for decades. Homologues of the Goose Replikin appeared from 1917 to 2006 in strains including each responsible for the three pandemics of 1918, 1957, and 1968 in H1N1, H2N2 and H3N2 with further substitutions between H1N2, H7N7, H5N2 and H5N1. Even certain substitutions which have occurred in the Goose Replikin tend to be selective and retained for years, rather than random. Thus despite the common assumption that amino acid substitutions should occur at random, it would appear that not all substitutions in influenza are, in fact, random. This Replikin conservation over decades allows the production of synthetic influenza vaccines which rapidly and inexpensively can be prepared in advance and can be effective for more than one year.

Therefore a target for synthetic influenza vaccines is the conserved Replikin Scaffold in influenza virus. A Replikin Scaffold comprises a series of conserved peptides comprising a sequence of about 16 to about 30 amino acids and further comprising
(1) a terminal lysine and an optional lysine in the residue portion immediately adjacent to the terminal lysine;
(2) a terminal histidine and another histidine in the residue portion immediately adjacent to the terminal histidine;
(3) at least one lysine within about 6 to about 10 amino acid residues from at least one other lysine; and
(4) at least about 6% lysines within the 16 to about 30 amino acid peptide.
Replikin Scaffold peptide also refers to an individual member or a plurality of members of a series of a Replikin Scaffold.

A non-limiting and preferred target for synthetic influenza vaccines is an influenza virus Replikin Scaffold comprising a sequence of about 29 amino acids and a lysine immediately adjacent to the terminal lysine. In another preferred target, a leucine five amino acid residues from the terminal histidine of the Replikin Scaffold is substituted with another amino acid. In another preferred target, substitution of a leucine is made with a hydrophobic amino acid. In another preferred target, substitution of a leucine is made with a methionine or an isoleucine.

A less-preferred target for synthetic influenza vaccine may be an Exoskeleton Scaffold in a first strain of influenza virus comprising a first peptide of about 29 amino acids and
(1) a terminal lysine and a lysine immediately adjacent to the terminal lysine;
(2) a terminal histidine and a histidine immediately adjacent to the terminal histidine;
(3) no lysine within 6 to 10 amino acid residues from any other lysine wherein an earlier-arising specimen of the first strain or another strain of virus comprises a Replikin Scaffold of about 29 amino acids.

In Table 1, the history of the Goose Replikin and its homologues are tracked from 1917 to the present outbreak of avian H5N1 virus. Table 1 demonstrates conservation of the "scaffold" homology of the Goose Replikin in virulent strains of influenza.

Table 1 illustrates the history, by year or smaller time period, of the existence in the protein structure of the Goose Replikin and its homologues in other influenza Replikins. Table 1 further illustrates the history of amino acid substitutions in those homologues and the conservation of certain amino acids of the Replikin structure which are essential to the definition of a Replikin and the function of rapid replication supplied by Replikins.

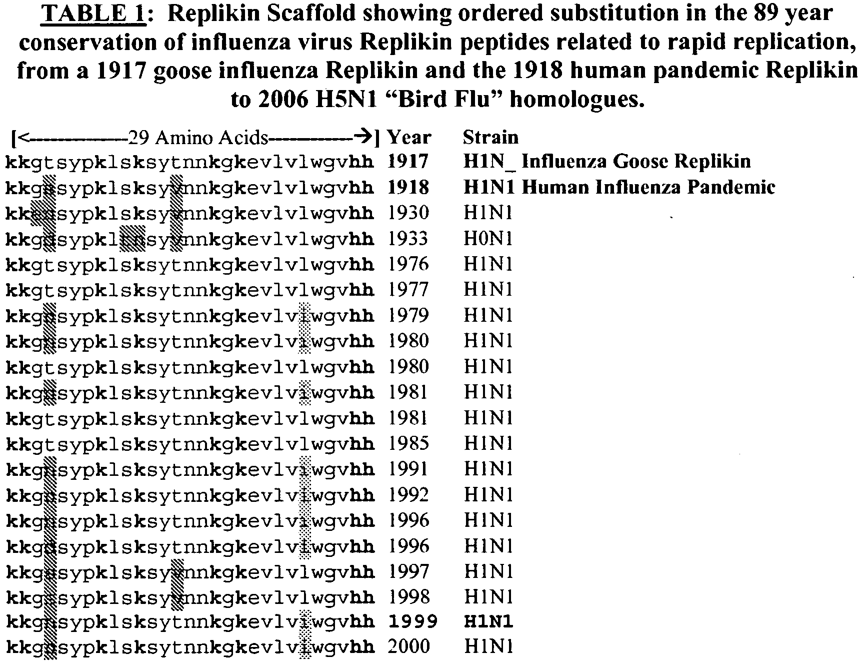

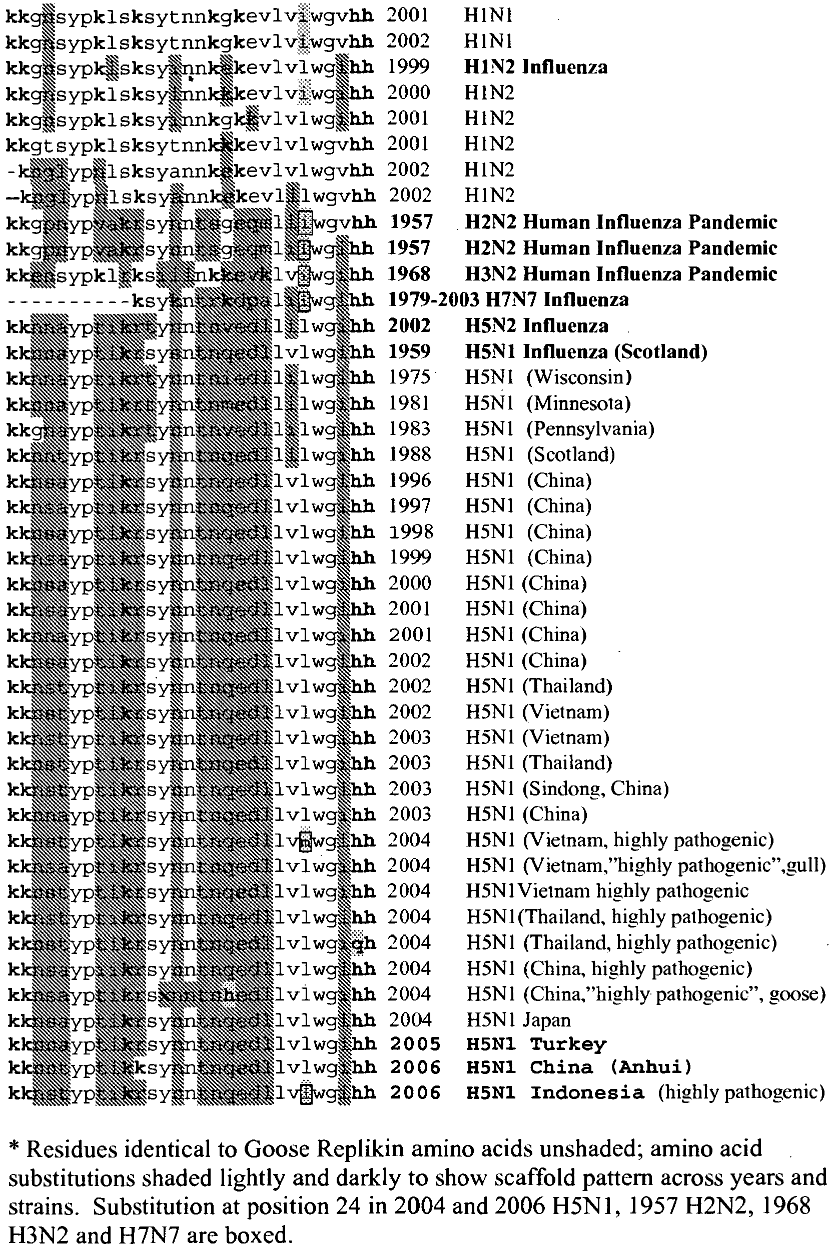

A review of Table 1 illustrates that if random substitution of amino acids were to occur in virulent strains of influenza from 1917 through the present, certain framework amino acids of the Goose Replikin would not be conserved from year to year in strains in which epidemics occurred. However, contrary to what would result from random substitution, virulent strains of influenza from year to year consistently contain conserved amino acids at those positions that define a Replikin. That is, if a substitution were to occur in one of the amino acids that define a Replikin, e.g., a lysine or a histidine, the definition of the Replikin would be lost. Nevertheless, the Replikin sequence is conserved over more than 85 years. Thus, since there is conservation of certain amino acids over decades, substitution cannot be said to be completely at random. The fact that substitutions do occur in amino acids that are not essential to the definition of a Replikin (i.e., amino acids other than lysines or histidines) demonstrates the importance of the Replikin in the pathogenicity of the strain.

It may be further noted from Table 1 that when substitutions do occur, they are seen to occur at certain apparently preferred positions of the Replikin Scaffold. Table 1 illustrates recurring substitutions at positions 1, 3-24 and 26-27. Further, while substitutions occur throughout these positions, a lysine continues to exist at a position 6 to 10 amino acids from the second lysine (which has not been substituted in these virulent strains).

Even when there is a substitution of a lysine position within the 29 amino acid stretch, as is seen in 1957, when K at position 11 shifts to position 10, that new position has been maintained until 2005, as have YP, AY, N (position 15), and LVLWG to conserve the homologous structure of the Replikin Scaffold with few exceptions.

It is important to note that an extra K has appeared in the Replikin Scaffold of a 2006 strain of H5N1 in China (Anhui). This presence of an extra K signals an increase in the Replikin count within the Replikin Scaffold. The 2006 China (Anhui) strain has a Replikin count of 6.6 (as discussed below). A Replikin count of 6.6 is the highest ever observed for an H5N1 strain and is comparable in the entire A strain of influenza only to the Replikin count of the influenza strain that caused the 1918 Pandemic. If this initial 2006 report is repeated and maintained, it may indicate that the Counts of 4.5 and 4.0 in 2004 and 2005 respectively will be substantially increased, and foretell a continuing or increased epidemic of H5N1 "Bird Flu."

Earlier virulent strains of influenza virus containing Replikin Scaffold sequences have been reviewed to determine effect on replication rate and virulence when a Replikin Scaffold degenerates into a non-Replikin sequence but maintains identifiable homology to the scaffold structure of the Replikin Scaffold. It has been found that loss of internal Ks and Hs in the Replikin scaffold (as distinct from those at the end), causing it no longer to represent for example fewer than the 2 or 3 included replikins within the 29 amino acids, or even to no longer to be a replikin at all, but preserving the outer "shell," e.g. but not limited to 29 amino acids KKXXXXXXXXXXXXXXXXXXXXXXXXXHH of the scaffold, is found frequency in virus sequences of isolates losing their high replikin count and their virulence typically at the end of an epidemic or outbreak. This "destruction" (or incomplete synthesis) of the replikin scaffold, going along as it does with a decline in Replikin Count. is therefore useful as another index of the failure of an outbreak or epidemic, just as the appearance of the fully represented scaffold, with internal Ks and Hs, producing more replikins and thus a higher Replikin Count, which appearance usually occurs when the Replikin Count exceeds 3 and is headed up, is useful as an index of the coming appearance of the outbreak or epidemic. In addition to this practical use, this shell phenomenon is another "proof of principle" evidence that the replikin scaffold is a critical structural element of the virus, not before recognized, having a function intimately associated with viral "success" at outbreaks and epidemics.

For example, an earlier-arising specimen of a particular influenza species having a Replikin Scaffold is later changed as follows:
1) The length of 29 amino acids is preserved;
2) The first two amino acid positions (1 and 2) are preserved, i.e. KK;
3) The last two amino acid positions (28 and 29) are preserved, i.e. HH;
4) But there is no longer a K 6 to 10 amino acids from KK (needed for the definition of a Replikin).
As such, the Scaffold is no longer a Replikin Scaffold, but now is a Scaffold Exoskeleton. While Replikin Scaffolds are associated with high Replikin Counts and the occurrence of epidemics, Scaffold Exoskeletons are associated with virus dormancy and the reduction or end of the epidemic. Thus Scaffold Exoskeletons appear to be degenerative structures left as residues when Replikin Scaffolds and specific viral outbreaks are declining, thus a useful diagnostic structure for this purpose. This confirms the revelation and use of Replikin Scaffolds as 1) targets for anti-rapid replication agents such as antibodies or small inhibitory RNAs and 2) the basis of anti-viral vaccines.

The integrity and conservation of the Replikin Scaffold may be seen by the fact that there is preferably a fixed 29 amino acid sequence that begins with two lysines and ends with two histidines. Less preferably the scaffold is 16 to about 30 amino acids in length.

### Amino Acid Substitutions

Substitutions in Replikin Scaffolds that do not destroy the scaffold structure appear unrestricted to particular amino acids. However, because certain amino acids share common chemical features that have a similar effect on the structure and function of a peptide, a substitution of one amino acid sharing a similar structural effect on the Replikin sequence with a similar amino acid is postulated to be more likely than the substitution of an amino acid by another amino acid from a different group.

Amino acids may be grouped in four categories, acid, basic, hydrophilic and hydrophobic. It is postulated that an amino acid in a particular group would be more likely substituted with an amino acid within its group than an amino acid in a different group. The acid group, having an acidic side chain, includes aspartic acid and glutamic acid. The basic group, having a basic side chain, includes histidine, lysine and arginine. The neutral hydrophilic group, having a polar neutral side chain, includes asparagine, glutamine, tyrosine, threonine, serine and cysteine. The hydrophobic group, having a non-polar neutral side chain, includes methionine, tryptophan, phenylalanine, isoleucine, alanine, glycine, proline, valine and leucine. As such, a preferred substitution of a particular amino acid in a Replikin Scaffold sequence, as contemplated within the current invention, would be a substitution within the same amino acid group as the substituted amino acid. For example, it is postulated that a leucine would more likely be substituted by a methionine than by a glutamic acid and substitution of a methionine would be more likely not to destroy some function of a particular portion of a peptide.

### H5N1 Influenza Conservation of Replikin Scaffold

There has been concern that the current outbreak of high mortality H5N1 "bird flu" in several countries may represent the first phase of an overdue influenza pandemic. A report (Ungchusak K et al.N Eng J Med 2005 Jan 27; 352(4):323-5) suggests that in the first probable person-to-person transmission of H5N1, "sequencing of the viral genes identified no change in the receptor-binding site of hemagglutinin or other key features of the virus. The sequences of all eight viral gene segments clustered closely with other H5N1 sequences from recent avian isolates in Thailand." Phylogenetic analysis suggested that from the absence of evidence of "reassortment with human influenza viruses" that H5N1 is not a new variant. However, we now report three recent changes in a specific H5N1 protein sequence at sites which had not been changed in the last two H5N1 epidemics and in fact had been conserved since 1959. Previously, there has been no protein chemistry which correlated with virus epidemics and dormancy. We found that each of the three influenza pandemics of the last century, H1N1, H2N2 and H3N2, retrospectively was predicted by and correlated with an increase in the concentration of a specific class of peptides in the virus, rich in lysine and histidine, associated with rapid replication, called Replikins. We have now again found the Replikins to be predictive in each of the three H5N1 epidemics, in 1997, 2001, and 2003-2004 (Figure 11). Each year that they appear in isolates, the Replikins can now be counted per 100 amino acids as in Figure 11, and their sequences analyzed and compared as in Table 1. Analysis of Replikins may be accomplished manually or in a preferred aspect of the present invention automatically by software for the purpose of counting Replikin concentration in available sequence information.

A graph illustrating a rapid increase in the concentration of Replikin patterns in the hemagglutinin protein of the H5N1 strain of influenza prior to the outbreak of three "Bird Flu" epidemics may be seen in Figure 11. A review of Figure 11 illustrates that an increasing Replikin concentration ('Replikin Count') in the hemagglutinin protein of H5N1 preceded three 'Bird Flu' Epidemics. For example, an increase in the Replikin Count (Means+/-SD) in 1995 to 1997 preceded the Hong Kong H5N1 epidemic of 1997 (E1). An increase in the Replikin Count from 1999 to 2001 preceded the epidemic of 2001 (E2). And an increase in Replikin Count from 2002 to 2004 preceded the epidemic in 2004 (E3). The decline in 1999 occurred with the massive culling of poultry in response to the E1 epidemic in Hong Kong.

In addition to the total number of Replikins in the virus protein, the structure of each Replikin through time is informative and provides targets for vaccines and other therapies such as antibodies and small RNA's. As discussed above, Table 1 shows a Replikin first observed in a goose infected with influenza in 1917 (Goose Replikin). Constant length, constant lysines at the amino terminal and histidine residues at the carboxy terminal were conserved in different strains in a fixed scaffold for decades. Homologues of the Goose Replikin appeared from 1917 to 2006 in strains including each responsible for the three pandemics of 1918, 1957, and 19681, H1N1, H2N2 and H3N2, and with further substitutions between H1N2, H7N7, H5N2 and H5N1. Even certain substitutions which have occurred in the Goose Replikin tend to be selective and retained for years, rather than random. Thus despite the common assumption that amino acid substitutions should occur at random, it would appear that not all substitutions in influenza are, in fact, random. This Replikin conservation over decades allows the production of synthetic influenza vaccines which rapidly and inexpensively can be prepared in advance and can be effective for more than one year.

In the 1997 H5N1 Hong Kong epidemic, the human mortality rate was approximately 27%. In 2004, of the fifty-two people reported to have been infected by H5N1 in Asia approximately 70% died. Most recently, nine of the eleven cases in Vietnam from December 28, 2004 to January 27, 2005 died. Although the virulence of the virus appears to have increased, any changes thought to be required for further spread from human to human, had been thought not yet to have occurred. However, we now have observed recent substitutions in three H5N1 Replikin amino acid residues at position numbers 18, 24 and 28 of the Goose Replikin scaffold from isolates in Vietnam, Thailand and China in 2004 (see Table 1). Substitution at site number 24 has not occurred since the appearance of H5N1 in 1959 but was present in the last two influenza pandemics caused by other strains, H2N2 in 1957 and H3N2 in 1968, together responsible for over two million human deaths, and in a recent virulent epidemic caused by H7N7 (see Table 1). While these are only hints of possible danger, these data on substitution, combined with the rising Replikin Count shown in Figure 11, and the past correlation of such Replikin data with pandemics, does not give the same reassurance as that obtained from phylogenetic analysis that the virus is unlikely to spread human to human.

Table 1 demonstrates the integrity of the Replikin Scaffold in virulent strains of influenza. As discussed above, degeneration of the Replikin Scaffold into an Exoskeleton Scaffold is seen to decrease pathogenicity. The integrity and conservation of the Replikin Scaffold, therefore, is seen by the fact that there is generally a fixed 29 amino acid sequence that begins with two lysines and ends with two histidines.

An aspect related to the present invention is a combination of Replikin structure and function to track the pathogenicity or rate of replication of a virus, epidemic or pandemic or to predict the occurrence of epidemics or pandemics. An example of this combination is the ability of the Replikin algorithm of the invention to be used to count increases in Replikin counts in influenza strains such as the strain of 1918 and the current H5N 1 strain of H5N 1. The Replikin Count of the 1918 influenza pandemic and the current outbreak of "Bird Flu" demonstrate the predictive capacity of this exemplary aspect in accordance with and made possible by the invention.

### Single substitution discovered in H5N1 strain in humans in Indonesia and Vietnam not present in previous H5N1 strains but historically present in most recent two killing human pandemics of 1957 (H2N2) and 1968 (H3N2)

A review of Table 1 above reveals that the H2N2 strain of influenza virus responsible for the high-mortality influenza pandemic of 1957 and the H3N2 strain of influenza responsible for the high mortality influenza pandemic of 1968 contain a single amino acid substitution at amino acid number 24 in the Goose Replikin Scaffold. See Table 1. Applicants have newly discovered, and herein report, that a recent single amino acid substitution in an H5N1 virus protein found in humans in Indonesia and Vietnam also has a substitution in the Goose Replikin at amino acid number 24. The H5N1 Goose Replikin peptide recently isolated from humans in Indonesia and Vietnam is KKNSTYPTIKRSYNNTNQEDLLV(M/I)WGIHH where the Indonesia strain of H5N1 contains an isoleucine at position 24 and the Vietnam strain of H5N1 contains a methionine at position 24. As was described above, Applicants teach that substitutions at amino acid 24 are predictive of increased virulence and human pandemics.

The Indonesian strain of H5N1 recently isolated in humans is significant since the substitution at amino acid number 24 in the Goose Replikin of H5N1 (here designated the "S" substitution) was absent from all earlier H5N1 strains but was present in the last two high-mortality influenza pandemics, 1957 (H2N2) and 1968 (H3N2). The pandemics of 1957 and 1968 were responsible for millions of deaths. The amino acid substitution at position 24 was likewise present in a recent (fortunately brief) outbreak of H7N7 with one human death. See Table 1. Nevertheless, the amino acid substitution at position 24 in the H5N1 Goose Replikin was absent from all analyzed and recorded isolates of H5N1 from 1959 up to the present (2006).

A review of the over 12 million entries at the National Library of Medicine (Pubmed) detected the S substitution in amino acid sequences and tracked the sequences in which S occurred through the years with the aid of FluForecast® software (see www.Replikins.com).

The S substitution was not observed in recent H5N1 isolates from chickens. Instead, the S substitution was observed only recently in H5N1 isolates from humans in cases with high mortality. The S substitution was observed in isolates from humans in Vietnam in 2004 and in Indonesia in 2006. See Table 1. Epidemiological evidence in humans in Vietnam and Indonesia suggests the S substitution in the H5N1 Goose Replikin correlates with person-to-person "clusters" where transmission from bird to human has not been established and human-to-human transmission has not been ruled out. The S substitution suggests human-to-human transmission of H5N1 may already have occurred, although infrequently to date.

World Health Organization and U.S. Centers for Disease Control spokespersons have recently announced that no "significant" worrisome sequence changes have been observed in H5N1 isolates from high mortality H5N1 Indonesian human cases. Significant sequence changes are thought by those of skill in the art to be required before person-to-person transmission may occur. As such, "significant" sequence changes are thought to be a necessary prerequisite for a human pandemic. The recent correlation of the S substitution to person-to-person "clusters" suggests the "significant" sequence changes expected by WHO and CDC prior to human-to-human transmission of H5N1 may be found, unexpectedly, in a single amino acid change within the Goose Replikin Scaffold as predicted above.

Despite the fact that the Goose Replikin virus peptide has been found to be highly conserved in H1N1, H2N2, H2N3, and H5N1, for 88 years (from 1917 to the present), a surprising mutation, the S mutation, has been identified within the Goose Replikin at a particularly conserved position, position 24. As has been established above, an increase in concentration of Replikins in proteins has been associated with rapid replication and epidemics. See, e.g., Figures 6, 7, 8, 10 and 11. A rising "Replikin Count" (number of Replikins per 100 amino acids) was found to be quantitatively correlated with and frequently predictive in advance of each major flu epidemic, the last three flu pandemics of the past century and the last three H5N1 epidemics from 1997 to the present. See Figures 6, 7, 8, 10 and 11.

A single substitution such as the S-substitution may, alone, not be the sole cause of a pandemic. Nevertheless, the occurrence of the S substitution has been a marker in the last two high-mortality pandemics, in 1957 and 1968. Further, the occurrence of the S substitution in H5N1 solely in humans and never in chickens (to date), accompanied by high Replikin Counts and high mortality rates, in total suggests that H5N1 may indeed be on the path to a human pandemic.

The evidence suggesting a higher likelihood of a forthcoming human pandemic contrasts markedly with previous more comforting assessments made prior to the identification of the S substitution in combination with high Replikin Counts and high mortality rates. As such, even while the detailed function of the S-substitution is not fully known, the evidence suggests the epidemiological function of the S-substitution in combination with increased Replikin Counts is apparent.

What finally determines if, and when, a full-force pandemic from H5N1 materializes is still unknown. Previous lack of information of the final substituted structure of the H5N1 that might be the agent of the next pandemic has stalled attempts to produce an appropriate vaccine. The herein disclosed Replikin peptide structures and the Goose Replikin Scaffold provide necessary direction for the production of a vaccine against a possible forthcoming pandemic.

### S-Substituted Replikin Scaffold as Target

A non-limiting example of a target for synthetic vaccines provided by predictive substitution in a Replikin Scaffold in an emerging strain of influenza virus is provided in Table 1 in the sequences labeled "1957 H2N2 Human Influenza Pandemic," "1968 H3N2 Human Influenza Pandemic," "1979-2003 H7N7 Influenza," 2004 H5N1 (Vietnam, highly pathogenic)" and "2006 H5N1 Indonesia (highly pathogenic)." As discussed above, as compared to the Goose Replikin and all other sequences disclosed in Table 1, the substitution of leucine with isoleucine in the 1957 and 1968 pandemic strains and with isoleucine in the 1979-2003 strain of H7N7 provides predictive weight that the recent substitution of leucine with methionine in an emerging strain of H5N1 in 2004 in Vietnam and with isoleucine in an emerging strain of H5N1 in 2006 in Indonesia will result in continued increases in virulence. The high mortality rate in Vietnam and Indonesia coupled with some evidence of human-to-human transmission suggest virulence has in fact increased in these substituted strains. The substituted Replikin Scaffold peptides of these strains, therefore, are non-limiting preferred embodiments of Replikin Scaffold peptides useful in development of a synthetic vaccine. Another non-limiting embodiment of the invention is the Replikin Scaffold peptides of the substituted H5N1 isolated from Vietnam in 2004 and the substituted H5N1 isolated from Indonesia in 2006 with the fifth position from the terminal histidine substituted with any amino acid residue other than leucine. In a more preferred embodiment, the Replikin Scaffold peptides are substituted at the fifth position from the terminal histidine with any hydrophobic amino acid other than leucine.

In another embodiment of the invention, scaffold peptides of the 1957 H2N2 pandemic strain, the 1968 H3N2 pandemic strain and the 1979-2003 H7N7 strain also provide homologous targets for synthetic vaccines against these emerging strains.

The present description, therefore, also provides an isolated Replikin peptide of the invention wherein the Replikin peptide comprises about 29 amino acids. The isolated Replikin peptide may comprise the amino acid sequence KKNSTYPTIKRSYNNTNQEDLLV[S]WGIHH wherein [S] may be any amino acid that is substituted as compared to a second strain of influenza virus otherwise comprising about the same Replikin peptide sequence. In a preferred embodiment, [S] may be a hydrophobic amino acid. In a still preferred embodiment, [S] may be a methionine or an isoleucine. In a further preferred embodiment, the above-embodied sequences are isolated from the H5N1 strain of influenza.

### VI. METHOD OF PREDICTING INCREASE IN INFLUENZA VIRULENCE USING REPLIKIN SCAFFOLDS

Historic data in Replikin Scaffolds over time provide tools for predicting the virulence of an emerging strain of influenza virus. If the strain of a strain of influenza virus is increasing in Replikin Concentration over time, it is an emerging strain of influenza virus and an increase in virulence is predictable. If an emerging strain of influenza virus comprises an amino acid substitution in a Replikin Scaffold peptide that is correlated with an increase in virulence or host mortality in a strain of influenza virus that has historically been highly virulent, the emerging strain may be predicted to have an increase in virulence.

The present invention therefore provides a method of predicting an increase in replication, virulence or host mortality in a first strain of influenza virus comprising
(1) identifying an influenza virus Replikin Scaffold comprising a plurality of Replikin Scaffold peptides wherein the Replikin Scaffold comprises a first Replikin Scaffold peptide isolated from the first strain of influenza virus, a second Replikin Scaffold peptide isolated from a second strain of influenza virus and a third Replikin Scaffold peptide isolated from a third strain of influenza virus,
(2) identifying an amino acid in the third Replikin Scaffold peptide that is substituted as compared to the second Replikin Scaffold peptide,
(3) determining that the third strain of influenza virus demonstrates increased replication, virulence or host mortality as compared to the second strain of influenza virus,
(4) determining that the amino acid residue substituted in the third Replikin Scaffold as compared to the second Replikin Scaffold peptide is in the same residue position as the amino acid residue substitution in the first Replikin Scaffold peptide as compared to the second Replikin Scaffold,
(5) comparing the Replikin Count of the first strain of influenza virus to the Replikin Count of earlier isolates of the first strain of influenza virus,
(6) determining that the Replikin Count of the first strain of influenza virus is greater than the Replikin Count of earlier arising isolates of the first strain of influenza virus,
(7) predicting an increase in replication, virulence or host mortality in the first strain of influenza virus as compared to the second strain of influenza virus.

For example, as discussed above and disclosed in Table 1, the substitution of leucine in the Goose Replikin and its homologues in the disclosed Replikin Scaffold with isoleucine in the 1957 and 1968 pandemic strains of H2N2 and H3N2 and with isoleucine in the 1979-2003 strain of H7N7 provides predictive weight that the recent substitution of leucine with methionine in an emerging strain of H5N1 in 2004 in Vietnam and with isoleucine in an emerging strain of H5N1 in 2006 in Indonesia will result in continued increases in virulence. As can be seen from the high mortality rates and evidence of coupling of transmission between humans, virulence in the Vietnam and Indonesia strains has apparently increased.

Therefore, in a non-limiting preferred embodiment of the method of the invention the Replikin Scaffold peptide of the first strain of influenza virus has undergone a substitution of a leucine for any other amino acids. In a more preferred embodiment, the substitution has been with any hydrophobic amino acid other than leucine. In a still more preferred embodiment, the substitution has been with a methionine or isoleucine. In yet another preferred embodiment, the substitution in the Replikin Scaffold peptide of the first strain of influenza virus is at position 24 of the Replikin Scaffold peptide or in the alternative at the fifth residue position from the terminal histidine.

The present description also provides a method for predicting pandemics comprising isolating and sequencing viral DNA, scanning the resulting sequence for encoded amino acid sequence and following the method of prediction described herein for amino acid sequences. A nucleic acid sequences is homologous with an amino acid sequence if it encodes the sequence of the amino acid. In a preferred embodiment of a method of the invention a method for predicting pandemics comprises scanning the resulting nucleic acid sequence, determining changes at position 24 of the Goose Replikin, and predicting a future pandemic based upon the presence of an S substitution in the Goose Replikin of H5N1.

The non-limiting method discussed above may be employed in any Replikin Scaffold series that has been discovered or that may hereinafter be disclosed.

### VII. METHOD OF PREDICTING INCREASED INFLUENZA VIRULENCE USING REPLIKINS

The present description also provides a non-limiting method of predicting an increase in replication, virulence or host mortality in a strain of influenza virus by monitoring changes in Replikin sequences over time, geography or epidemiological event. A method of predicting an increase in replication, virulence or host mortality in a strain of influenza virus comprises
(1) identifying a first strain of influenza virus in which the Replikin Count of the strain of influenza virus is greater than the Replikin Count of earlier arising isolates of the first strain of influenza virus
(2) identifying a first Replikin peptide in the first strain of influenza virus
(3) identifying a second Replikin peptide in a second strain of influenza virus that is homologous with the first Replikin peptide but for an amino acid substitution wherein the amino acid substitution is not made at histidine residues,
(4) identifying a third Replikin peptide in a third strain of influenza virus that is homologous with the second Replikin peptide but for an amino acid substitution at the same position as the amino acid substitution in the first Replikin peptide,
(5) determining that the third strain of influenza virus demonstrates increased replication, virulence or host mortality over the second strain of influenza virus,
(6) predicting that the first strain of influenza virus will demonstrate increased replication, virulence or host mortality over the second strain of influenza virus.

In a preferred embodiment of the method, the amino acid substitution is not made at a lysine residue or histidine residue. The Replikin algorithm must be satisfied in each of the first, second and third Replikin peptides.

The present description further provides an influenza Replikin peptide isolated from the H5N1 strain of influenza.

### VIII. HOMOLOGOUS SCAFFOLD SEQUENCE IN SHRIMP WHITE SPOT VIRUS

The inventors have further discovered that the White Spot Shrimp Virus has an exceptionally high Replikin Count as compared to all other viruses and organisms surveyed for Replikins up to the present time (with the exception of malaria). While Replikins have been shown to be essential accompaniments of rapid replication in fungi, yeast, viruses, bacteria, algae, and cancer cells, the inventors have provided the first demonstration of the presence of Replikins in marine organisms other than algae. And, as with algae, the presence of Replikins is again related to rapid infestations. In shrimp, the white spot virus has destroyed millions of dollars of harvest of shrimp, first in eastern countries, and now in western hemisphere countries. At present, there is no effective prevention or treatment. Other examples of Replikin high mortality marine viral disease have been demonstrated in fish such as carp and hemorrhagic disease in salmon, and are probably widespread in marine ecology and disease.

The presence of repeat sequences of the Replikins of the nucleocapsid protein of shrimp white spot syndrome virus (WSSV) accounts for the unusually high Replikin Count of 103.8. This virus Replikin Count is much higher than the Replikin Counts of for example influenza viruses which usually range from less than 1 up to 5 or 7, and is comparable only to the record Replikin Count (so far) observed in Plasmodium Falciparum (malaria) of 111. Interestingly, while the shrimp white spot syndrome organism is a virus, and the Pl. Falciparum is a trypanosome, both spend an essential part of their reproductive cycles in red blood cells, an unusual host cell whether in shrimp (white spot virus) or man (malaria), both are fulminating rapidly replicating diseases with high mortality rates of their hosts, and both appear to use the same methods of increasing their high Replikin Counts to such record highs, namely, Replikin Repeats and Replikin Overlap.

The inventors have also established a relationship between virulent influenza virus and white spot virus in the Replikin Scaffold portions of the viruses. No relationship between these two viruses has been suggested previously. Although there is extensive substitution, the applicants' finding of several short Replikins of the Shrimp White Spot Syndrome Virus demonstrate significant homologies to the influenza virus Replikin sequences, especially with regard to length and key lysine (k) and histidine (h) residues (Fixed Scaffold or Replikin Scaffold), suggesting that similar mechanisms of Replikin production are used in both virus groups.

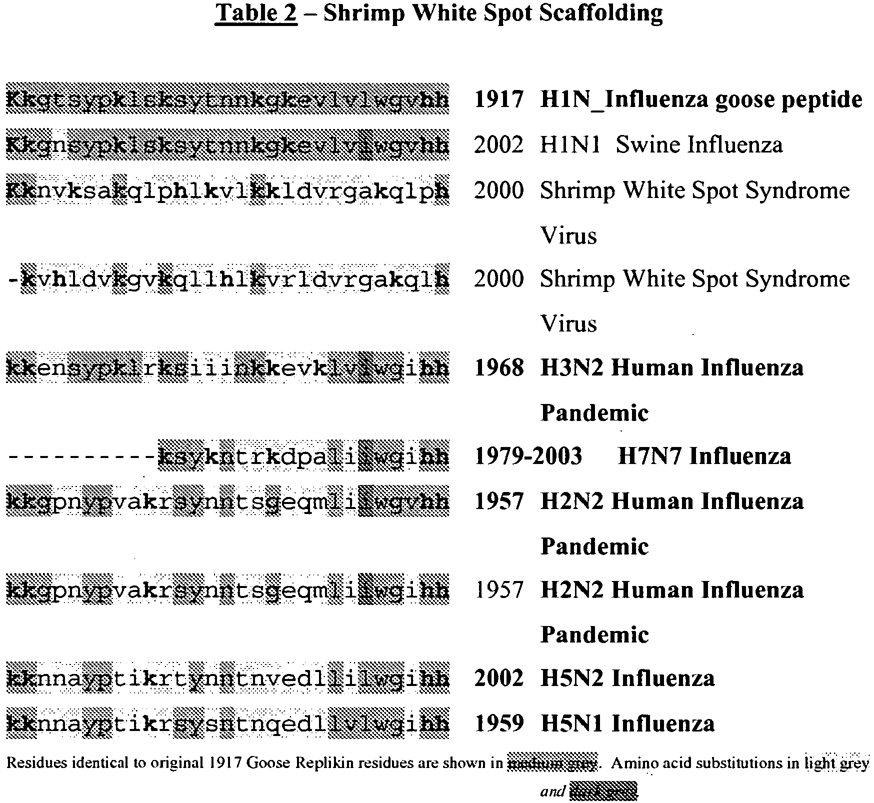

In addition, since many species, including but not limited to swine and birds, are known to provide animal "reservoirs" for human influenza infection, marine forms such as the shrimp virus can now be examined, with early warning diagnostic benefits possible for outbreaks such as swine flu and bird flu. While similarities of some influenza viruses were noted between species, and the transfer of these viruses interspecies was known, there was no previous quantitative method to gauge virus activity. It has not been possible previously to examine potential reservoirs for increased activity which might move into a different species; thus providing an advanced warning. The activity of the Replikins in each species can now be monitored constantly for evidence of increased viral replication rate and thus emergence of epidemics in that species which may then transfer to other species.

This data further supports the Replikins as a new class of peptides, with a history of its own, and a shared function of rapid replication and disease of its hosts. With the high mortality for its shrimp host, white spot syndrome virus can now have its Replikins examined as earlier forms of the virus Replikins, or as parallel morphological branches, which in either case may well act as reservoirs for bird and animal Replikins such as those in influenza viruses. The diagnostic and preventive uses of these Replikin findings in shrimp follow as they do in influenza and for other organisms containing Replikins.

### IX. HOMOLOGOUS SEQUENCES IN SARS VIRUS AND H3N2-FUJIAN INFLUENZA VIRUS REPLIKINS TRACED BACK TO A 1918 PANDEMIC

Certain SARS virus Replikin peptides share homology with peptides in several strains of influenza virus isolates including homology with Replikin Scaffold sequences that are homologous with the Goose Replikin. Homology extends from virulent influenza strains in 2006 back to a sequence in the strain of the 1918 influenza pandemic responsible for the deaths of over 20 million people.

The SARS coronavirus first appeared in the 2002-2003 influenza season. The dual origin in 2002 of SARS Replikins, from influenza GR and coronavirus Replikins (or from some unknown shared precursor) is suggested by the following events, all of which occurred in 2002: 1) a condensation for the first time in 85 years is seen in the GR-H1N2 Replikin sequence from 29 to 28 amino acids (Table 3)(A similar condensation was found in H3N2 Fujian from 29 to 27 amino acids in the current epidemic (Table 3)); 2) the Replikin count of GR-H1N2 showed a marked decline consistent with GR moving out of H1N2; 3) the Replikin count of coronavirus nucleocapsid proteins showed a marked increase; and 4) SARS coronavirus appeared in 2002-2003 with Replikins containing the following motifs: **'kkg'** and **'k-k'**, previously see**n** in GR 1918 and GR-H1N2 2001; **'k-kk', 'kk'** and **'kl'** seen in influenza GR-H1N2 2001; **'kk'** seen in the avian bronchitis coronavirus Replikin; and **'kk-kk-k', 'k-k', 'kk', 'kl'** and **'kt'** seen in the Replikin of porcine epidemic diarrhea coronavirus (Table 3).

Table 3.
Goose Replikin (GR) sequences in different influenza strains from 1917 to 2003;
SARS and H3N2-Fujian appearance 2002-2003.

Replikins related to the Goose Replikin: Replikin Virus or other organism
Continuous amino acid sequences | Length containing Replikin

Shared motif and/or position– shaded (Number (Complete Replikins
Amino acid substitutions– clear backgroundof amino except for Fujian strain)
'Condensed' indicates condensation of acids)
sequence length in H1N2 and H3N2-Fujian

SEQ.ID
NO.

kkgtsypklsksytnnkgkevlvlwgvhh | | 29 | 1917 H1N_ Influenza Goose Replikin (GR)
kkgssypklsksyvnnkgkevlvlwgvhh | 743 | 29 | 1918 GR in H1N1Human Influenza
kkgsnypvakgsynntsgeqmliiwgvhh | 744 | 29 | 1958 GR H2N2 Influenza
kkgpnypvakgsynntsgeqmliiwgvhh | 745 | 29 | 1964,1965,1968 GR in H2N2 Influenza
kkgtsypklsksytnnkgkevlvlwgvhh | | 29 | 1976,'77,'80,'81,'85 GR in H1N1 Influenza
kknsayptikrsynntnqedllvlwgihh | | 29 | 1996-2001 GR in H5N1 Influenza
kkgdsypklsksytnnkgkevlviwgvhh | 746 | 29 | 1996 GR in H1N1 Influenza
kkgssypklsksyvnnkgkevlvlwgvhh | 747 | 29 | 1997,1998 GR in H1N1 Influenza
kkgnsypkisksyinnkekevlvlwgihh | 748 | 29 | 1999 GR in H1N2 Influenza
kkgnsypklsksyinnkkkevlviwgihh | 749 | 29 | 2000 GR in H1N2 Influenza
kkgnsypklsksyinnkgkkvlvlwgihh | 750 | 29 | 2001 GR in H1N2 Influenza
kkgtsypklsksytnnkkkevlvlwgvhh | | 29 | 2001 GR in H1N2 Influenza
knglypnlsksyannkekevlilwgvhh | | 28 | 2002 GR in H1N2 Influenza (condensed)
khldaykttfpptepkkdkkkk | | 21 | 2002-3 Human SARS nucleocapsid protein
kkensypklrksiiinkkkevlviwgihh | 751 | 29 | 1968-2001 GR in H3N2 Influenza (complete)
kleykypalnvtmpnndkfdklyiwgvhh | 752 | 29 | 1996 H3N2 Fujian Influenza (incomplete)
kykypalnvtmpnnekfdklyiwgvhh | 753 | 27 | 2003 H3N2 Fujian(condensed, incomplete)
ktgnaklqrkkekknkrettlqqh | | 24 | Porcine epidemic diarrhea coronavirus
kkinspgpkfegsgvpdnenlktsqqh | | 27 | Avian bronchitis coronavirus
kknvksakqlphlkvlldvrgakqlph | 754 | 27 | 2000 shrimp white spot syndrome virus
kkinspgpkfegsgvpdnenlktsqqh | | 27 | Avian bronchitis coronavirus
khlrefvfknkdgflyvykk | 755 | 20 | 2002-3 Human SARS spike protein
kkgakllhkpivwh | 756 | 14 | 2002-3 Human SARS nucleocapsid protein
khlrefvfknkdgflyvykk | | 20 | 2002-3 Human SARS spike protein
kkgakllhkpivwh | | 14 | 2002-3 Human SARS nucleocapsid protein
keeldkyfknh | | 11 | 2002-3 Human SARS spike protein
kkgakllhk | | 9 | 2002-3 Human SARS envelope protein
kyrylrhgk | | 9 | 2002-3 Human SARS spike protein
khldayk | | 7 | 2002-3 Human SARS nucleocapsid protein
ksrgipikkgh | | 11 | Nipah virus, v-protein
ksrimpikkgh | | 11 | Hendra virus, v-protein
kkflnqfkhh | | 10 | Sindbis virus
kkkskkhkdk | | 10 | EEL leukemia
khhpkdnlik | | 10 | BRCA-1 breast cancer
khkrkkfrqk | | 10 | Ovarian cancer
kagvaflhkk | | 10 | Glioma Replikin
kihlisvkk | | 9 | Smallpox virus
krfilhakk | | 9 | HIV TAT protein
klisihek | | 8 | Smallpox virus

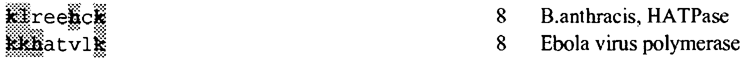

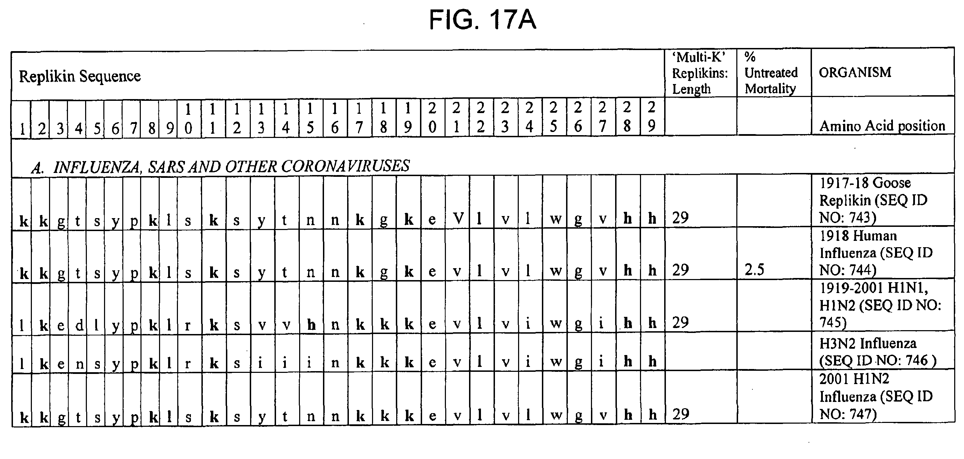

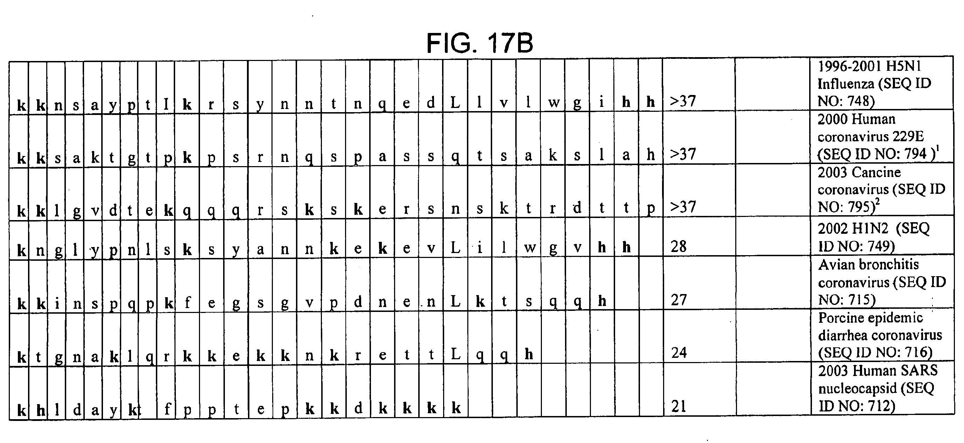

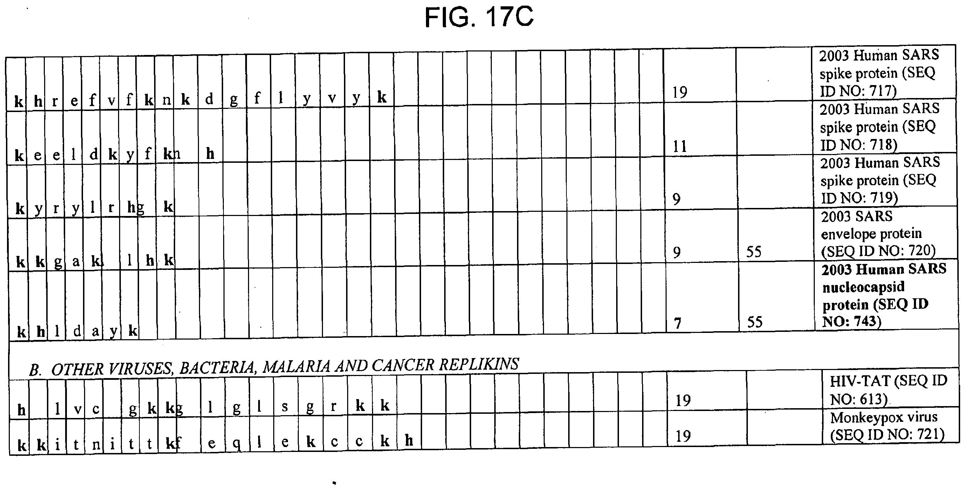

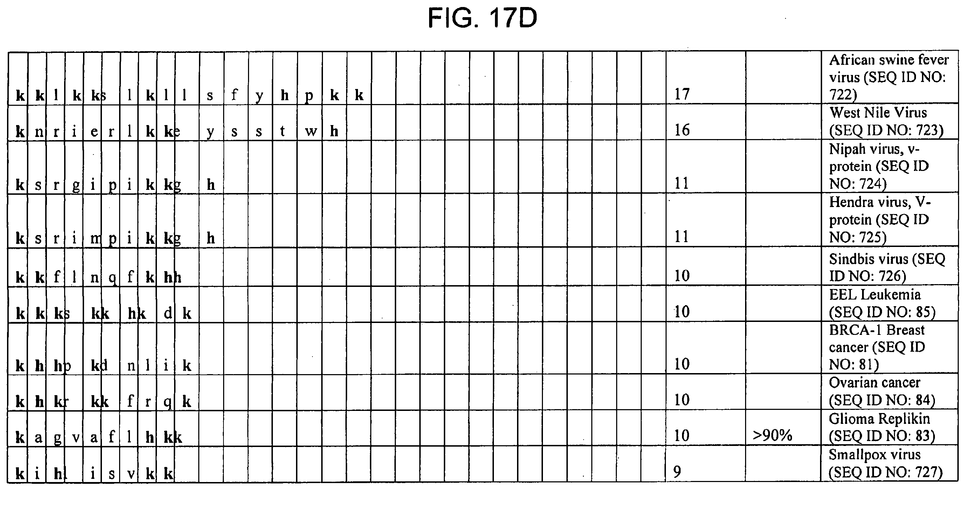

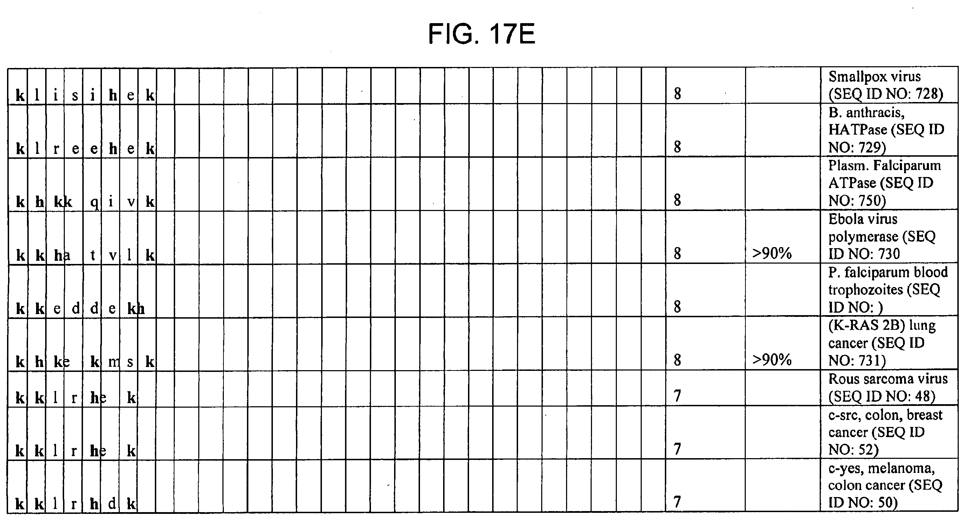

The recent increasingly high Replikin count peaks, including the presence of the 1917 Goose Replikin, now in H1N2 (Table 3), approaching the 1917 Replikin count, could be a warning of a coming pandemic which may already have begun since the SARS virus and the H3N2-Fujian virus are the current carriers of the short Replikin derivatives of the Goose Replikin seen (Figure 17) to be associated with high mortality.

Since the Goose Replikin has at least an 85 year history involving most or all of the A-strains of influenza and SARS, it and its components are conserved vaccine candidates for pan-strain protection. Condensed short SARS Replikins, 7 to 21 amino acids long, enriched in % lysine and histidine compared to the Goose Replikin, occurred in association with the higher mortality rate of SARS (10-55%) when compared to that (2.5%) of the Goose Replikin, 29 amino acids long. Short Replikins here mixed with long Replikins in SARS may be responsible for high mortality. This is also the case for Replikins of other organisms such as the ebola and smallpox viruses and anthrax bacteria (Table 3). These short SARS Replikins showed surprising homology with short Replikins of other organisms such as smallpox, anthrax, and ebola which are associated with even higher untreated mortality rates (Table 3). The detection of Replikins in SARS coronavirus homologous with influenza Replikins permitted the synthesis of small SARS antigens for vaccines.

Short synthetic vaccines can be much more rapidly produced at less expense and should avoid the side effects attendant on the contamination and the immunological interference engendered by multiple epitopes of thousands of undesired proteins in current whole virus vaccines in general. The short glioma Replikin 'kagvaflhkk' proved to be a successful basis for a synthetic *See* U.S. Patent No. 6,242,578. It produced anti-malignin antibody, which is cytotoxic to cancer cells at picograms/cell and relates quantitatively to the survival of cancer patients. Wesynthesized five SARS short Replikins, found in nucleocapsid, spike, and envelope proteins. See Example 6. We found that these synthetic short SARS Replikins when injected into rabbits also produced abundant specific antibody. For example, the 21 amino acid SARS nucleocapsid Replikin antibody binds at dilutions greater than 1 in 204,800. Because of previous unsuccessful attempts by others to achieve with various small peptides a strong immune response without the unwanted side effects obtained with a whole protein or the thousands of proteins or nucleic acids as in smallpox vaccine, the ability of small synthetic Replikin antigens to achieve strong immune responses is significant for the efficacy of these SARS vaccines.

We examined the relationship of Replikin structure in influenza and SARS viruses to increased mortality, with results as shown in Figure 17. The relation of high mortality to short or condensed Replikin sequences is seen in the high mortality organisms shown in Section B of Figure 17, in viruses other than influenza and SARS, and in bacteria, malaria and cancer. In support of the unifying concept of Replikin structure and of the relation of Replikins to rapid replication rather than any cell type or infectious organism, in addition to the prevalence of the basic Replikin structure in a broad range of viral, bacterial, malarial and cancer organisms in which replication is crucial to propagation and virulence, the following homologous sequences have been observed: note the "k"s in positions 1 and 2, note the alignment of "k"s as they would present to DNA, RNA or other receptor or ligand for incorporation or to stimulate rapid replication, note the frequency of "double k"s and "multiple k"s , note the frequency of "g" in position 3 and the occurrence of the triplets "kkg", "hek", "hdk" and "hkk" in the most condensed shortened Replikins associated with the highest mortality organisms, cancer cells and genes as diverse as the smallpox virus, the anthrax virus, Rous sarcoma virus and glioblastoma multiforme (glioma), c-src in colon and breast cancer, and c-yes in melanoma and colon cancer. Note also the almost identical Replikin structure for two recently emerging high mortality viruses in Australia and Southeast Asia, Nipah and Hendrah viruses. These two viruses are reported to have similar or identical antibodies formed against them but no structural basis has been known for this up till now, with our finding of their two almost identical Replikins, for this similar antibody.

Table 3 also shows the relationship of five SARS Replikins of 2003 which we have found both to the influenza Goose Replikin of 1917 and to two coronaviruses, the avian bronchitis coronavirus and the porcine epidemic diarrhea virus. The first 2003 human SARS Replikin in Table 3 and Figure 17 shows certain sequence homologies to the influenza virus goose 1917 and human 1918 Replikins through an intermediary structure of influenza H1N2 in 2002 (e.g., see Replikin "k" in positions 1, 18 and 19). The 1917 Goose Replikin sequence is seen in Table 3 and Figure 17 to have been largely conserved despite many substitutions in amino acids which are not crucial to the definition of Replikins through 1999 (substitutions are shown in italics). The original 29 amino acid 1917 Replikin sequence was then found to have been almost exactly restored to its structure of 1917-1918 in the 2001 H1N2 Replikin. However, the 2002 H1N2 influenza Replikin has been shortened from 29 to 28 amino acids and the "shift to the left" of amino acids kevl(i/v)wg (v/i)hh is clearly evident.

In 2003, one Replikin was further shortened (or compacted) to the 21 amino acid Replikin of the first listed 2003 human SARS virus. The % k of the 2003 SARS Replikin is now 38.1 % (8/21) in comparison to 20.7 % of the Goose Replikin and the 1918 Human Pandemic Replikin. Compared to the influenza 29 amino acid Replikin, three SARS Replikins were found to be further shortened (or compacted) to 19, 11 and 9 amino acid long sequences, respectively. In the SARS 9 amino acid sequences shown, the % k is 44.4% (4/9). With the shortening of the SARS Replikin, the SARS mortality rate in humans rose to 10% in the young and 55.5 % in the elderly compared to the 2.5 % mortality in the 1918 influenza pandemic.

The amino acid sequences are shown in Table 3 to emphasize the degree of homology and conservation for 85 years (1917-2002) of the influenza Replikin, for which evidence has first been observed in the 1917 Goose Replikin. No such conservation has ever been observed before. Table 3 also illustrates that the Replikins in the 2003 human SARS virus, in addition to having homologies to the influenza Replikins which first appeared as the 1917 Goose Replikin and the 1918 Human Pandemic influenza Replikin, show certain sequence homologies to both the coronavirus avian bronchitis virus Replikin (e.g. "k" in positions 1 and 2, end in "h") and to the coronavirus acute diarrhea virus Replikin (e.g. "k" in positions 1 and 11, "h" at the end of the Replikin). This evidence of relation to both influenza and coronavirus Replikins is of interest because SARS arose in Hong Kong as did several recent influenza epidemics and earlier pandemics, and the SARS virus has been classified as a new coronavirus partly because of its structure, including nucleocapsid, spike, and envelope proteins. Certain epidemiological evidence also is relevant in that SARS made its first appearance in humans as the epidemic pneumonia, which erupted, in a crowded Hong Kong apartment house where there was a severe back-up of fecal sewage, which was airborne by ventilating fans.

### X. INFLUENZA VACCINES, TREATMENTS AND THERAPEUTICS

The present invention also provides a vaccine comprising any one or more of the isolated sequences described above, or any antigenic subsequence of any one or more of the isolated sequences described above, or an antibody that binds to any of the isolated sequences described above or their subsequences

Currently, vaccine formulations for influenza are changed twice yearly at international WHO and CDC meetings. Vaccine formulations are based on serological evidence of the most current preponderance of influenza virus strain in a given region of the world. However, prior to the present invention there has been no correlation of influenza virus strain specific amino acid sequence changes with occurrence of influenza epidemics or pandemics.

The observations of specific Replikins and their concentration in influenza virus proteins provides the first specific quantitative early chemical correlates of influenza pandemics and epidemics and provides for production and timely administration of influenza vaccines tailored specifically to treat the prevalent emerging or re-emerging strain of influenza virus in a particular region of the world. By analyzing the protein sequences of isolates of strains of influenza virus, such as the hemagglutinin protein sequence, for the presence, concentration and/or conservation of Replikins, influenza virus pandemics and epidemics can be predicted. Furthermore, the severity of such outbreaks of influenza can be significantly lessened by administering an influenza peptide vaccine based on the Replikin sequences found to be most abundant or shown to be on the rise in virus isolates over a given time period, such as about one to about three years.

An influenza peptide vaccine of the invention may include a single Replikin peptide sequence or may include a plurality of Replikin sequences observed in influenza virus strains. Preferably, the peptide vaccine is based on Replikin sequence(s) shown to be increasing in concentration over a given time period and conserved for at least that period of time.

For example, a preferred vaccine may include a Replikin sequence that is a member of a Replikin Scaffold. The highly conserved nature of Replikin Scaffolds in combination with the correlation of Replikin Scaffold sequences with highly virulent strains of influenza virus make Replikin Scaffold sequence are preferred sequence for a synthetic vaccine. A member of the Goose Replikin Scaffold has proven effective in providing a strong immune response when administered to rabbits and chickens subcutaneously. *See* Example 7.

A vaccine of the invention may include a conserved Replikin peptide(s) in combination with a new Replikin(s) peptide or may be based on new Replikin peptide sequences. The Replikin peptides can be synthesized by any method, including chemical synthesis or recombinant gene technology, and may include non-Replikin sequences, although vaccines based on peptides containing only Replikin sequences are preferred. Preferably, vaccine compositions of the invention also contain a pharmaceutically acceptable carrier and/or adjuvant.

The vaccines of the present invention can be administered alone or in combination with antiviral drugs, such as gancyclovir; interferon; interleukin; M2 inhibitors, such as, amantadine, rimantadine; neuraminidase inhibitors, such as zanamivir and oseltamivir; and the like, as well as with combinations of antiviral drugs.

The vaccine of the present invention may be administered to any animal capable of producing antibodies in an immune response. For example, the influenza vaccine of the present invention may be administered to a rabbit, a chicken, a pig or a human. A vaccine of the present invention may be directed at a range of strains of influenza or a specific strain of influenza.

In a non-limiting aspect in accordance with the present invention, a vaccine may be directed to an immune response against animal or human strain of influenza including influenza B, (A)H1N1, (A)H2N2, (A)H3N2, H5N1 or any human variant of the virus that may arise hereafter, as well as strains of influenza predominantly in animals such as the current avian H5N1. An influenza vaccine may further be directed to a particular Replikin amino acid sequence in any portion of an influenza protein. The influenza vaccine comprising a Replikin Scaffold of the H5N1 virus KKNSTYPTIKRSYNNTNQEDLLVLWGIHH covalently linked to the UTOPE KKKKHKKKKH and the well known keyhole limpet cyanin adjuvant has provided a strong immune response in chickens and rabbits. See Example 7.

A non-limiting embodiment of the invention provides a vaccine comprising the Replikin Scaffold sequence KKNSTYPTIKRSYNNTNQEDLLV[S]WGIHH wherein [S] may be any amino acid that is substituted as compared to a second strain of influenza virus otherwise comprising about the same Replikin peptide sequence. In a preferred embodiment, [S] may be a hydrophobic amino acid. In a still preferred embodiment, [S] may be a methionine or an isoleucine. In a further preferred embodiment, the above-embodied sequences are isolated from the H5N1 strain of influenza.

In a further non-limiting aspect, an influenza vaccine may comprise a UTOPE such as KKKKH or KKKKHKKKKKH. In a further alternative, a vaccine may comprise the addition of an adjuvant such as the well known keyhole limpet hemocyanin herein denoted -KLH. In yet a further preferred non-limiting aspect, an influenza vaccine may comprise a Replikin Scaffold of influenza H5N1 further comprising two UTOPES and an adjuvent sequence such as KKNSTYPTIKRSYNNTNQEDLLVMWGIHH KKKKHKKKKKHK-KLH or KKNSTYPTIKRSYNNTNQEDLLVIWGIHH KKICKHKKKKICHK-KLH. An aspect of the present invention may comprise a Replikin Scaffold sequence previously isolated and shown in Table 1 as one of the Bird Flu Replikins labelled"2004 H5N1 Vietnam, highly pathogenic" or "2006 Indonesia, highly pathogenic.

With administration of 100 ug of the peptide of vaccine injected subcutaneously into rabbits and chickens an antibody response would be expected from unvaccinated dilutions of less than 1:50 to reach a peak in the third to fourth week after vaccination of from a dilution of 1:120,000 to greater than 1:240,000.

Synthetic Replikin vaccines, based on Replikins such as the glioma Replikin "kagvaflhkk" or the hepatitis C Replikin "hyppkpgcivpak", or HIV Replikins such as "kcfncgkegh" or "kvylawvpahk" or preferably, an influenza vaccine based on conserved and/or emerging or re-emerging Replikin(s) over a given time period may be used to augment antibody concentration in order to lyse the respective virus infected cells and release virus extracellularly where chemical treatment can then be effective.

It is preferable to utilize only the specific Replikin structure when seeking to induce antibodies that will recognize and attach to the Replikin fragment and thereby cause destruction of the cell. Even though the larger protein sequence may be known in the art as having a "replication associated function," vaccines using the larger protein often have failed or proven ineffective.

Although the present inventors do not wish to be held to a single theory, the studies herein suggest that the prior art vaccines are ineffective, that is fail to produce an adequate immune response in the form of antibodies and/or cellular immunity, and are not protective, because they are based on the use of the larger protein sequence. The larger protein sequence invariably has one or more epitopes (independent antigenic sequences that can induce specific antibody formation); Replikin structures usually comprise one of these potential epitopes. The presence of other epitopes within the larger protein may interfere with adequate formation of antibodies to the Replikin, by "flooding" the immune system with irrelevant antigenic stimuli that may preempt the Replikin antigens, See, e.g., Webster, R.G., J. Immunol., 97(2):177-183 (1966); and Webster et al., J. Infect. Dis., 134:48-58, 1976; Klenerman ct al, Nature 394:421-422 (1998) for a discussion of this well-known phenomenon of antigenic primacy whereby the first peptide epitope presented and recognized by the immune system subsequently prevails and antibodies are made to it even though other peptide epitopes are presented at the same time. This is another reason that, in a vaccine formulation, it is important to present the constant Replikin peptide to the immune system first, before presenting other epitopes from the organism so that the Replikin is not pre-empted but lodged in immunological memory.

### Quantitative Measurement Early Response(s) to Replikin Vaccines

The ability to measure quantitatively the early specific antibody response in days or a few weeks to a Replikin vaccine is a major practical advantage over other vaccines for which only a clinical response months or years later can be measured.

### Adjuvants

Various adjuvants may be used to enhance the immunological response, depending on the host species, including but not limited to Freund's (complete and incomplete), mineral gels, such as aluminum hydroxide, surface active substances such as lysolecithin, pluronic polyols, polyanions, peptides, oil emulsions, key limpet hemocyanin, dintrophenol, and potentially useful human adjuvants such as BCG and Corynebacterium parvum. In addition to the use of synthetic UTOPEs as vaccines in themselves, UTOPEs can be used as adjuvants to other Replikin vaccines and to non-Replikin vaccines.

### UTOPES

It has been demonstrated that high % k Replikins generate the highest antibody responses when administered to rabbits. These synthetic peptides, designed by the inventors, are designated as Universal synthetic epitopes, or "UTOPE's" , and the vaccines based upon these UTOPEs, are designated "UVAX"s. UVAXs, deduced synthetic vaccines, may be used as sole vaccines or as adjuvants when administered with more specific Replikin vaccines or other vaccines. The following are examples of deduced UTOPEs and UVAXs:

| | |
|---|---|
| DEVISED SYNTHETIC REPLIKIN | SEQ ID NO: |
| (UTOPE OR UVAX) | |
| KKKKHK | _ |
| KKKHKK | _ |
| KKHKKK | _ |
| KHKKKK | _ |
| KKKKKKH | _ |
| KKKICKHK | _ |
| KKKKHKK | _ |
| KKKHKKK | _ |
| KKHKKKK | _ |
| KHKKKKK | _ |
| HKKKICKK | _ |

Recognin and/or Replikin peptides may be administered to a subject to induce the immune system of the subject to produce anti-Replikin antibodies. Generally, a 0.5 to about 2 mg dosage, preferably a 1 mg dosage of each peptide is administered to the subject to induce an immune response. Subsequent dosages may be administered if desired.

### Identification of Substitutions Provides Early Development of Vaccine

Previous egg and cell-based methods for production of influenza virus vaccines contain thousands of unwanted proteins which may produce undesirable side effects, take 6 to 9 months or more to make, and more time to test. FluForecast® now permits advance strain-specific warning of 1 to 3 years that an epidemic or pandemic is on its way, and the discovery of the key Replikin and the substituted amino acid "S" permits more accurate, potentially safer, synthetic vaccines to be prepared promptly, within a few days, for testing.

Identifying the S-substitution in the Goose Replikin in H5N1 provides the skilled artisan with a target for the development of a vaccine against a future human pandemic resulting from an S-substitution in H5N1. A vaccine may comprise any portion of the sequence KKNSTYPTIKRSYNNTNQEDLLV[S]WGIHH where the S-substitution at position 24 (five residues from the terminal histidine) may represent any amino acid substitution. In a preferred embodiment the S-substitution represents an amino acid other than leucine. In a more preferred embodiment, the S-substitution represents any a hydrophobic amino acid other than leucine. In a still more preferred embodiment, the Substitution represents methionine or isoleucine. One non-limiting vaccine embodiment comprises from 7 to 29 amino acids of the sequence KKNSTYPTIKRSYNNTNQEDLLV[S]WGIHH. In another non-limiting embodiment, the vaccine comprises from 7 to 29 amino acids of the sequence KKNSTYPTIKRSYNNTNQEDLLVLWGIHH. The vaccine may also alternatively comprise an adjuvant such as key limpet hemocyanin or any other adjuvant and/or a UTOPE such as KKKKHK or any other UTOPE. In another non-limiting embodiment, an antibody to an a 7 to 29 amino acid antigenic subsequence of the sequence KKNSTYPTIKRSYNNTNQEDLLVLWGIHH is contemplated. Any antigenic subsequence having about 7 to about 29 amino acids of a Replikin Scaffold or Replikin sequence of the invention is contemplated for vaccines and stimulation of the immune system to produce antibodies.

An antibody to all or any portion of the sequence KKNSTYPTIKRSYNNTNQEDLLV[S]WGIHH may be developed by one of skill in the art. An antibody particularly directed at an epitope including position 24 may likewise be developed by one of skill in the art. In a preferred embodiment the S-substitution represents an amino acid other than leucine. In a more preferred embodiment, the S-substitution represents any hydrophobic amino acid other than leucine. In a still more preferred embodiment, the S-substitution represents methionine or isoleucine. An antibody to all or any portion of the sequence including, for example, the sequence KKNSTYPTIKRSYNNTNQEDLLV(M/I) may be developed by one of skill in the art.

Antibodies to a Goose Replikin sequence having an S substitution may be used for treatment of infection from H5N1 with antibodies to the sequence or to diagnose virulent infections of H5N1 in birds, animals or humans.

All Replikin sequences that have to date been tested to date have demonstrated immunogenic properties. Injection of the 16-mer Replikin from malignin into rabbits produced specific antibody to the 16-mer Replikin. Example 6 and Figures 9A and 9B of U.S. 6,242,578 B1. SARS Replikins found in nucleocapsid, spike, and envelope proteins of the SARS coronavirus were synthesized and tested on rabbits. An immune response was quantified. *See* Example 6. A 41 amino acid Replikin sequence KKNSTYPTIKRSYNNTNQEDLLVLWGIHHKKKKHKKKKKHK-KLH designated Vaccine V120304U2 was designed by the inventors from the 29 amino acid Replikin Scaffold of H5N1 "Bird Flu" Influenza Replikins labeled "2004 H5N1 Vietnam, highly pathogenic" in Table 1 with the addition of two UTOPE units (KKKKHK) on the C-terminal end of the H5N1 scaffold and an additional adjuvant (keyhole limpet hemocyanin (denoted -KLH)) covalently linked on the C-terminal end of the two UTOPE units. 100 ug of Vaccine V120304U2 was injected subcutaneously into rabbits and chickens. The antibody response was measured before vaccination and at from one week after injection to eight weeks after injection. An antibody response was noted at one week and reached a peak in the third to fourth week after vaccination. Peak antibody responses ranged from a dilution of 1:120,000 to a dilution of greater than 1:240,000. One of skill in the art may develop an antibody to each of the Replikin sequences and Replikin Scaffold Sequences discussed herein.

### Replikin Nucleotide Sequences

Replikin DNA or RNA may have a number of uses for the diagnosis of diseases resulting from infection with a virus, bacterium or other Replikin encoding agent. For example, Replikin nucleotide sequences may be used in hybridization assays of biopsied tissue or blood, e.g., Southern or Northern analysis, including in situ hybridization assays, to diagnose the presence of a particular organism in a tissue sample or an environmental sample, for example. The present invention also contemplates kits containing antibodies specific for particular Replikins that are present in a particular pathogen of interest, or containing nucleic acid molecules (sense or antisense) that hybridize specifically to a particular Replikin, and optionally, various buffers ard/or reagents needed for diagnosis.

Also related to the invention are oligoribonucleotide sequences that include antisense RNA and DNA molecules and ribozymes that function to inhibit the translation of Replikin- or recognin-containing mRNA. Both antisense RNA and DNA molecules and ribozymes may be prepared by any method known in the art. The antisense molecules can be incorporated into a wide variety of vectors for delivery to a subject. The skilled practitioner can readily determine the best route of delivery, although generally i.v. or i.m. delivery is routine. The dosage amount is also readily ascertainable.

Particularly preferred antisense nucleic acid molecules are those that are complementary to a Replikin sequence contained in a mRNA encoding, for example, an influenza virus polypeptide, wherein the Replikin sequence comprises from 7 to about 50 amino acids including (1) at least one lysine residue located six to ten residues from a second lysine residue; (2) at least one histidine residue; and (3) at least 6% lysine residues. More preferred are antisense nucleic acid molecules that are complementary to a Replikin present in the coding strand of the gene or to the mRNA encoding the influenza virus hemagglutinin protein, wherein the antisense nucleic acid molecule is complementary to a nucleotide sequence encoding a Replikin that has been demonstrated to be conserved over a period of six months to one or more years and/or which are present in a strain of influenza virus shown to have an increase in concentration of Replikins relative to Replikin concentration in other influenza virus strains. The increase in Replikin concentration preferably occurs over a period of at least six months, preferably about one year, most preferably about two or three years or more.

Similarly, antisense nucleic acid molecules that are complementary to mRNA those that are complementary to a mRNA encoding Replikins comprising a Replikin sequence of from 7 to about 50 amino acids including (1) at least one lysine residue located six to ten residues from a second lysine residue; (2) at least one histidine residue; and (3) at least 6% lysine residues. Also preferred are nucleic acid molecules that are complementary to mRNA encoding a Replikin Scaffold. More preferred are antisense nucleic acid molecules that are complementary to the codingstrand of the gene or to the mRNA encoding a protein of the virus.

### XI. COMPUTER

The present invention also provides methods for predicting an increase in virulence in an emerging strain of influenza virus using a computer. Data banks comprising nucleotide and/or amino acid sequences can also be scanned by computer for the presence of influenza strains containing Replikins and Replikin Scaffold peptides meeting the requirements for predicting an emerging strain of influenza virus will have increased virulence.

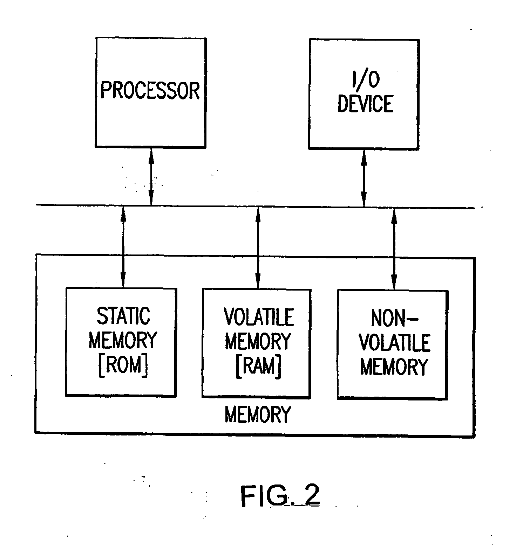

Figure 2 is a block diagram of a computer available for use with the foregoing embodiments of the present invention. The computer may include a processor, an input/output device and a memory storing executable program instructions representing the virulence predicting methods of the foregoing embodiments. The memory may include a static memory, volatile memory and/or a nonvolatile memory. The static memory conventionally may be a read only memory ("ROM") provided on a magnetic, or an electrical or optical storage medium. The volatile memory conventionally may be a random access memory ("RAM") and may be integrated as a cache within the processor or provided externally from the processor as a separate integrated circuit. The non-volatile memory may be an electrical, magnetic or optical storage medium.

The following examples are for illustration of the present invention only and do not limit the full scope of the invention provided herein. It will be appreciated that modifications and variations of the present invention are encompassed by the above teachings and within the purview of the appended claims without departing from the intended scope of the invention.

### EXAMPLE 1

### Process for Extraction, Isolation and Identification of Replikins and the Use of Replikins to Target, Label or Destroy Replikin-Containing Organisms

### a) Algae

The following algae were collected from Bermuda water sites and either extracted on the same day or frozen at -20 degrees C and extracted the next day. The algae were homogenized in a cold room (at 0 to 5 degrees C) in 1 gram aliquots in neutral buffer, for example 100 cc. of 0.005M phosphate buffer solution, pH 7 ("phosphate buffer") for 15 minutes in a Waring blender, centrifuged at 3000 rpm, and the supernatant concentrated by perevaporation and dialyzed against phosphate buffer in the cold to produce a volume of approximately 15 ml. The volume of this extract solution was noted and an aliquot taken for protein analysis, and the remainder was fractionated to obtain the protein fraction having a pK range between 1 and 4.

The preferred method of fractionation is chromatography as follows: The extract solution is fractionated in the cold room (4° C) on a DEAE cellulose (Cellex-D) column 2.5x11.0 cm, which has been equilibrated with 0.005M phosphate buffer. Stepwise eluting solvent changes are made with the following solutions:
Solution 1- 4.04 g. NaH2P04 and 0.5g NaH2P04 are dissolved in 15 liters of distilled water (0.005 molar, pH 7);
Solution 2 - 8.57 g. NaH2P04 is dissolved in 2,480 ml. of distilled water;
Solution 3 - 17.1 g. of NaH2P04 is dissolved in 2480 ml of distilled water (0.05 molar, pH 4.7);
Solution 4 - 59.65 g. of NaH2P04 is dissolved in 2470 ml distilled water (0.175 molar);
Solution 5 - 101.6 g. of NaH2P04 is dissolved in 2455 ml distilled water (pH 4.3);
Solution 6 - 340.2 g. of NaH2P04 is dissolved in 2465 of distilled water (1.0 molar, pX-i 4.1);
Solution 7 - 283.63 g. of 80% phosphoric acid (H3P04) is made up in 2460 ml of distilled water (1.0 molar, pH 1.0).

The extract solution, in 6 to 10 ml volume, is passed onto the column and overlaid with Solution 1, and a reservoir of 300 ml of Solution 1 is attached and allowed to drip by gravity onto the column. Three ml aliquots of eluant are collected and analyzed for protein content at OD 280 until all of the protein to be removed with Solution 1 has been removed from the column. Solution 2 is then applied to the column, followed in succession by Solutions 3, 4, 5, 6 and 7 until all of the protein which can, be removed with each Solution is removed from the column. The eluates from Solution 7 are combined, dialyzed against phosphate buffer, the protein content determined of both dialysand and dialyzate, and both analyzed by gel electrophoresis. One or two bands of peptide or protein of molecular weight between 3,000 and 25,000 Daltons are obtained in Solution 7. For example the algae Caulerpa mexicana, Laurencia obtura, Cladophexa prolifera, Sargassum natans, Caulerpa verticillata, Halimeda tuna, and Penicillos capitatus, after extraction and treatment as above, all demonstrated in Solution 7 eluates sharp peptide bands in this molecular weight region with no contaminants. These Solution 7 proteins or their eluted bands are hydrolyzed, and the amino acid composition determined. The peptides so obtained, which have a lysine composition of 6% or greater are Replikin precursors. These Replikin peptide precursors are then determined for amino acid sequence and the Replikins are determined by hydrolysis and mass spectrometry as detailed in U.S. Patent 6,242,578 B1. Those that fulfill the criteria defined by the " 3-point-recognition" method are identified as Replikins. This procedure can also be applied to obtain yeast, bacterial and any plant Replikins.

### b) Virus

Using the same extraction and column chromatography separation methods as above in a) for algae, Replikins in virus-infected cells are isolated and identified.

### c) Tumor cells in vivo and in vitro tissue culture

Using the same extraction and column chromatography separation methods as above in a) for algae, Replikins in tumor cells are isolated and identified. For example, Replikin precursors of Astrocytin isolated from malignant brain tumors, Malignin (Aglyco 1OB) isolated from glioblastoma tumor cells in tissue culture, MCF7 mammary carcinoma cells in tissue culture, and P3J Lymphoma cells in tissue culture each treated as above in a) yielded Replikin precursors with lysine content of 9.1%, 6.7%, 6.7%, and 6.5% respectively. Hydrolysis and mass spectrometry of Aglyco 1OB as described in Example 10 U.S. 6,242,578 B1 produced the amino acid sequence, ykagvaflhkkndiide the 16-mer Replikin.

### EXAMPLE 2:

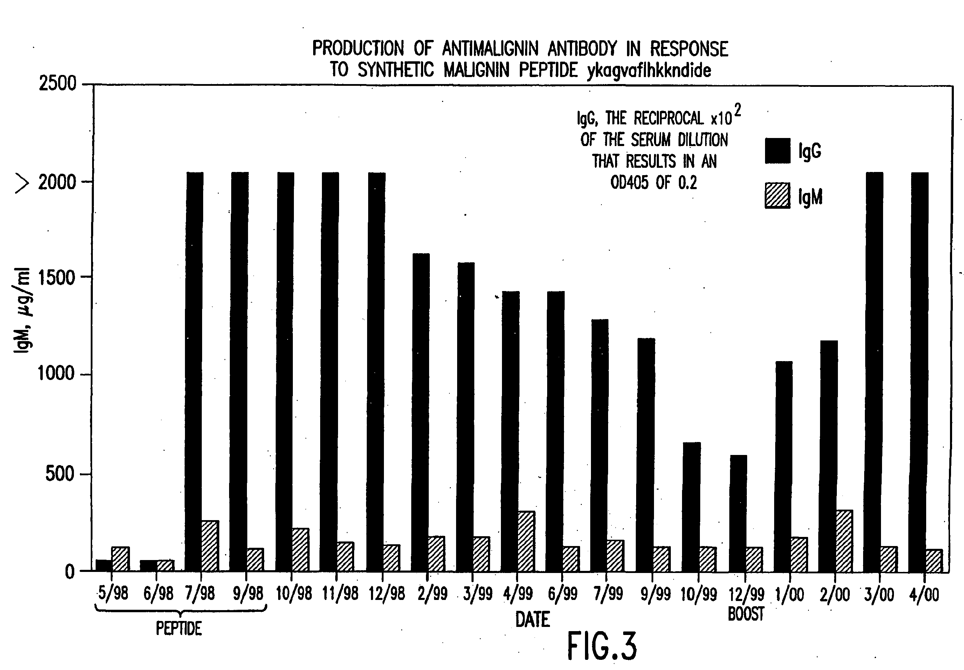

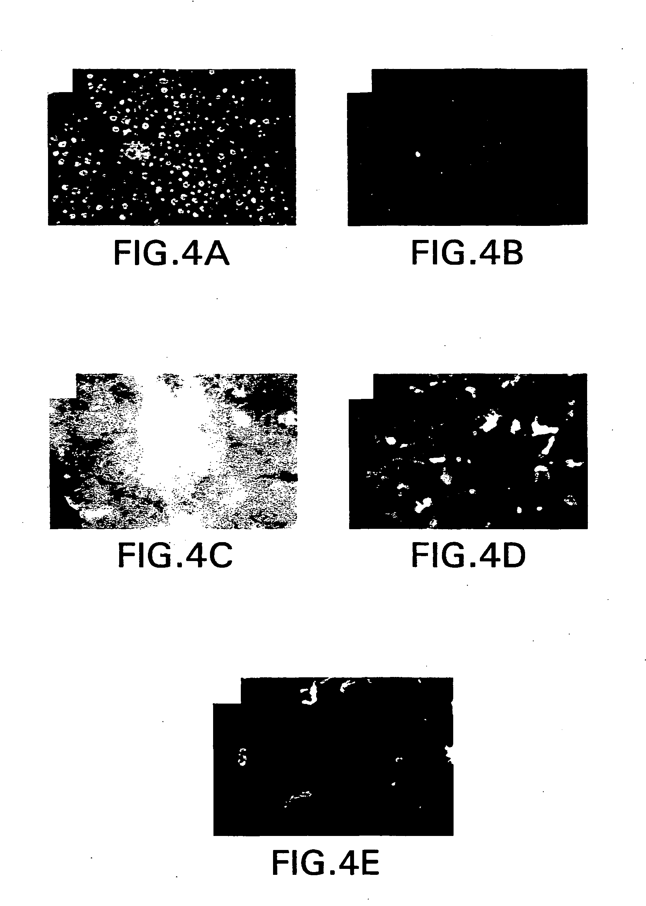

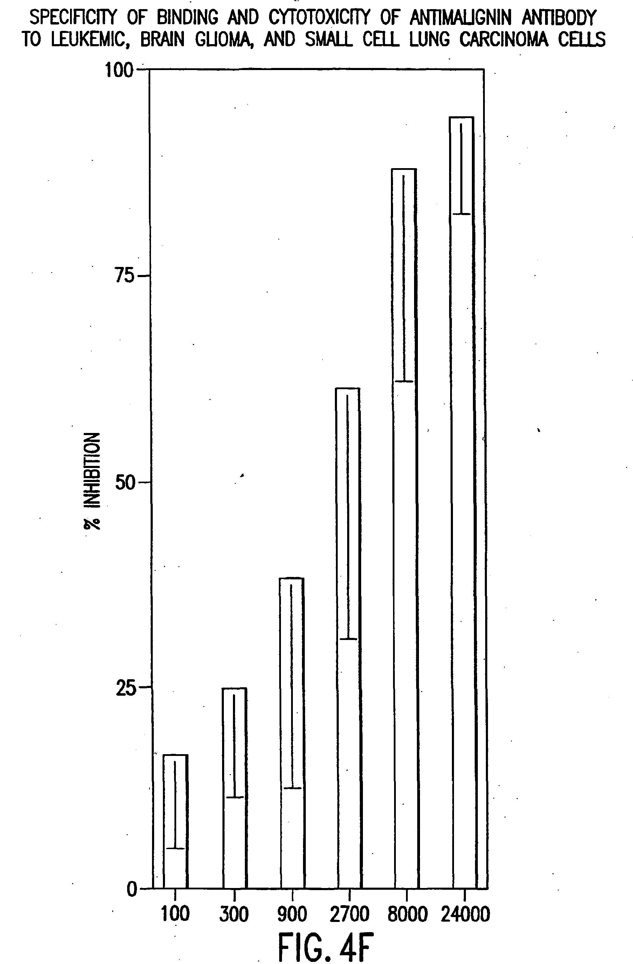

As an example of diagnostic use of Replikins: Aglyco 1OB or the 16-mer Replikin may be used as antigen to capture and quantify the amount of its corresponding antibody present in serum for diagnostic purposes are as shown in Figures 2, 3, 4 and 7 of U.S. 6,242,578 B1.

As an example of the production of agents to attach to Replikins for labeling, nutritional or destructive purposes: Injection of the 16-mer Replikin into rabbits to produce the specific antibody to the 16-mer Replikin is shown in Example 6 and Figures 9A and 9B of U.S. 6,242,578 B1.

As an example of the use of agents to label Replikins: The use of antibodies to the 16-mer Replikin to label specific cells which contain this Replikin is shown in Figure 8 and Example 6 of U.S. 6,242,578 B1.

As an example of the use of agents to destroy Replikins: The use of antibodies to the 16-mer Replikin to inhibit or destroy specific cells which contain this Replikin is shown in Figure 9 of U.S. 6,242,578 B1.

### EXAMPLE 3

Analysis of sequence data of isolates of influenza virus hemagglutinin protein or neuraminidase protein for the presence and concentration of Replikins is carried out by visual scanning of sequences or through use of a computer program based on the 3-point recognition system described herein. Isolates of influenza virus are obtained and the amino acid sequence of the influenza hemagglutinin and/or neuraminidase protein is obtained by any art known method, such as by sequencing the hemagglutinin or neuraminidase gene and deriving the protein sequence therefrom. Sequences are scanned for the presence of new Replikins, conservation of Replikins over time and concentration of Replikins in each isolate. Comparison of the Replikin sequences and concentrations to the amino acid sequences obtained from isolates at an earlier time, such as about six months to about three years earlier, provides data that are used to predict the emergence of strains that are most likely to be the cause of influenza in upcoming flu seasons, and that form the basis for seasonal influenza peptide vaccines or nucleic acid based vaccines. Observation of an increase in concentration, particularly a stepwise increase in concentration of Replikins in a given strain of influenza virus for a period of about six months to about three years or more is a predictor of emergence of the strain as a likely cause of influenza epidemic or pandemic in the future.

Peptide vaccines or nucleic acid-based vaccines based on the Replikins observed in the emerging strain are generated. An emerging strain is identified as the strain of influenza virus having the highest increase in concentration of Replikin sequences within the hemagglutinin and/or neuraminidase sequence during the time period. Preferably, the peptide or nucleic acid vaccine is based on or includes any Replikin sequences that are observed to be conserved in the emerging strain. Conserved Replikins are preferably those Replikin sequences that are present in the hemagglutinin or neuraminidase protein sequence for about two years and preferably longer. The vaccines may include any combination of Replikin sequences identified in the emerging strain.

For vaccine production, the Replikin peptide or peptides identified as useful for an effective vaccine are synthesized by any method, including chemical synthesis and molecular biology techniques, including cloning, expression in a host cell and purification therefrom. The peptides are preferably admixed with a pharmaceutically acceptable carrier in an amount determined to induce a therapeutic antibody reaction thereto. Generally, the dosage is about 0.1 mg to about 10 mg.

The influenza vaccine is preferably administered to a patient in need thereof prior to the onset of "flu season." Influenza flu season generally occurs in late October and lasts through late April. However, the vaccine may be administered at any time during the year. Preferably, the influenza vaccine is administered once yearly, and is based on Replikin sequences observed to be present, and preferably conserved in the emerging strain of influenza virus. Another preferred Replikin for inclusion in an influenza vaccine is a Replikin demonstrated to have re-emerged in a strain of influenza after an absence of one or more years.

### EXAMPLE 4

Analysis of sequence data of isolates of coronavirus nucleocapsid, or spike, or envelope, or other protein for the presence and concentration of Replikins is carried out by visual scanning of sequences or through use of a computer program based on the 3-point recognition method described herein.

### EXAMPLE 5

Analysis of sequence data of isolates of Plasmodium falciparum antigens for the presence and concentration of Replikins is carried out by visual scanning of sequences or through use of a computer program based on the 3-point recognition method described herein.

### EXAMPLE 6

Amino acid sequences of five short SARS Replikins found in nucleocapsid, spike, and envelope proteins of the SARS coronavirus were synthesized and tested on rabbits to test immune response to Replikin sequences in the SARS coronavirus. The following Replikin sequences were tested: (1) 2003 Human SARS nucleocapsid (SEQ ID NO: _); (2) 2003 Human SARS spike protein (SEQ ID NO: _); (3) 2003 Human SARS spike protein (SEQ ID NO: _); 2003 Human SARS spike protein; (SEQ ID NO: _); (4) 2003 SARS envelope protein (SEQ ID NO: _); and (5) 2003 Human SARS nucleocapsid protein (SEQ ID NO: _). Each synthesized peptide was injected subcutaneously into a rabbit. The tested rabbits produced measurable specific antibody to each of the five sequences that bound at dilutions of greater than 1 in 10,0000. The 21 amino acid SARS nucleocapsid Replikin antibody (SEQ. ID NO: _) was demonstrated to bind at dilutions greater than 1 in 204,800. Because of previous unsuccessful attempts by others to achieve with various small peptides a strong immune response without the unwanted side effects obtained with a whole protein or the thousands of proteins or nucleic acids as in smallpox vaccine, the ability of small synthetic Replikin antigens to achieve strong immune responses was shown to be significant for the efficacy of SARS vaccines.

### EXAMPLE 7

A 41 amino acid Replikin sequence KKNSTYPTIKRSYNNTNQEDLLVLWGIHHKKKKHKKKKKHK-KLH designated Vaccine V120304U2 was designed by the inventors from the 29 amino acid Replikin Scaffold of H5N1 "Bird Flu" Influenza Replikins labeled "2004 H5N1 Vietnam, highly pathogenic" in Table 1 with the addition of two UTOPE units (KKKKHK) on the C-terminal end of the H5N1 scaffold and an additional adjuvant (keyhole limpet hemocyanin (denoted -KLH)) covalently linked on the C-terminal end of the two UTOPE units. 100 ug of Vaccine V120304U2 was injected subcutaneously into rabbits and chickens. The antibody response was measured before vaccination and at from one week after injection to eight weeks after injection. An antibody response was noted at one week and reached a peak in the third to fourth week after vaccination. Peak antibody responses ranged from a dilution of 1:120,000 to a dilution of greater than 1:240,000. Antibody titers were determined with an enzyme linked immunosorbent assay (ELISA) with Peptide-GGG (goat gamma globulin) bound in solid phase (0.1 ug/100 ul/well) on high binding 96 well plates. The serum was first diluted 50 fold and then further diluted in 2-fold serial dilutions. The ELISA titer result was determined from the estimated dilution factor that resulted from an optical density at 405 nm of 0.2 and derived from nonlinear regression analysis of the serial dilution curve. Detection was obtained using a horse radish peroxidase conjugated secondary antibody and ABTS substrate (ABTS is a registered trademark of Boehringer Mannheim. GmbH). Results from tests on two chickens and two rabbits are provided in Table 4. Individual well results from the test on rabbit D4500 are provided in Table 5. In combination with the results reported in Example 6, in a total of six tests of Replikin sequences for antibody responses in rabbit or chicken, all six sequences provided a measurable antibody response and have proved antigenic.

**Table 4**

| Chickens injected with 100 µg V120304U2 on day 1. ELISA titer of antibody production on day 18 | | |
|---|---|---|
| Animal | Bleed Day | ELISA Titer |
| U0682 (Control) | Prior to administration of vaccine | <50 |
| u0682 | 18 days after administration | >204,800 |
| U0683 (Control) | Prior to administration of vaccine | <50 |
| u0683 | 18 days after administration | >204,800 |

| Rabbits injected with 100 µg V120304U2 on day 1. ELISA titer of antibody production on day 20 | | |
|---|---|---|
| Animal | Bleed Day | ELISA Titer |
| D4500 (Control) | Prior to vaccine administration | <50 |
| d4500 | 20 days after administration | >204,800 |
| D4501 (Control) | Prior to vaccine administration | 100 |
| d4501 | 20 days after administration | >204,800 |

**Table 5**

| Rabbits injected with 100 µg V120304U2 on day 1. OD450 results for titers on days 7, 20 and 28 in individual wells | | | | | | | |
|---|---|---|---|---|---|---|---|
| **Animal** | **Test Day** | Well 1 | Well 2 | Well 3 | Well 4 | Well 5 | Well 6 |
| **d4500** | Day 7 | 0.11 | 0.10 | 0.09 | 0.08 | 0.07 | 0.07 |
| | Day 20 | 0.49 | 0.38 | 0.23 | 0.19 | 0.22 | 0.17 |
| | Day 28 | 2.77 | 1.41 | 0.92 | 0.56 | 0.43 | 0.42 |

| | | Well 7 | Well 8 | Well 9 | Well 10 | Well 11 | Well 12 |
|---|---|---|---|---|---|---|---|
| **d4500** | Day 7 | 0.06 | 0.06 | 0.06 | 0.06 | 0.6 | 0.6 |
| | Day 20 | 0.02 | 0.16 | 0.17 | 0.15 | 0.19 | 0.28 |
| | Day 28 | 0.17 | 0.14 | 0.12 | 0.11 | 0.11 | 0.10 |

| | | Well 1 | Well 2 | Well 3 | Well 4 | Well 5 | Well 6 |
|---|---|---|---|---|---|---|---|
| **d4501** | Day 7 | 0.25 | 0.18 | 0.15 | 0.11 | 0.09 | 0.08 |
| | Day 20 | 0.50 | 0.23 | 0.20 | 0.16 | 0.18 | 0.18 |
| | Day 28 | 1.75 | 0.84 | 0.61 | 0.50 | 0.34 | 0.35 |

| | | Well 7 | Well 8 | Well 9 | Well 10 | Well 11 | Well 12 |
|---|---|---|---|---|---|---|---|
| **d4501** | Day 7 | 0.07 | 0.07 | 0.07 | 0.06 | 0.06 | 0.06 |
| | Day 20 | 0.16 | 0.18 | 0.16 | 0.17 | 0.17 | 0.25 |
| | Day 28 | 0.20 | 0.14 | 0.12 | 0.12 | 0.11 | 0.13 |

### EXAMPLE 8

Emergent virulent strains of influenza may be predicted using the following method. Analysis of sequence and epidemiological data of isolates of influenza virus for the presence of Replikin Scaffolds in virulent strains of influenza virus is carried out by visual scanning sequences in concert with epidemiological data or through use of a computer program based on the Replikin Scaffold algorithm described herein.

An isolated influenza virus peptide may be considered a Replikin Scaffold peptide if the isolated peptide comprises 16 to about 30 amino acids and further comprises
(1) a terminal lysine and optionally a lysine immediately adjacent to the terminal lysine.
(2) a terminal histidine and a histidine immediately adjacent to the terminal histidine,
(3) a lysine within about 6 to about 10 amino acids from another lysine; and
(4) at least 6% lysines.

Isolated sequences containing a Replikin Scaffold sequence are scanned for shared and substituted sequences with other members of the Replikin Scaffold series and compared by level of epidemiological virulence. Epidemiological virulence may be determined by extent of outbreak and/or by percent host mortality or by any method known to one of skill in the art.

Individual substitutions within individual Replikin Scaffold peptides are then scanned for a correlation to a highly virulent outbreak associated with the particular isolate from which the individual Replikin Scaffold peptide has been isolated. Current strains of influenza virus that have been shown to be emerging strains based on an increasing Replikin Count over 6 months to 3 years and that contain representative Replikin Scaffold peptides are scanned to determine if a substitutions correlated with increased virulence in earlier-arising strains is now present in the emerging strain of influenza virus. If an individual substitution correlating with increased virulence is present, a prediction may be made that the emerging strain of influenza virus will have increased virulence.

## Claims

1. A vaccine comprising the substantially isolated Replikin peptide comprising the amino acid sequence KKX₁X₂X₃YPTIKX₄X₅X₆NNTNX₇EDLLVX₈WGIX₉H (SEQ ID NO: 349) or an antigenic subsequence thereof, wherein
X₁ is N or G;
X₂ is N or S;
X₃ is A or T;
X₄ is R or K;
X₅ is S or T;
X₆ is any amino acid residue;
X₇ is any amino acid residue;
X₈ is isoleucine, glycine, alanine, valine, proline, phenylalanine, tryptophan, serine, threonine, tyrosine, cysteine, asparagine or glutamine; and
X₉ is H or Q.

2. The vaccine of claim 1, wherein X₆ is Y.

3. The vaccine of claim 1, wherein X₇ is Q, I, M, V or H.

4. The vaccine of claim 1, wherein X₇ is Q or H.

5. The vaccine of claim 4, wherein X₇ is Q.

6. The vaccine of claim 1, wherein X₈ is isoleucine, glycine, alanine, valine, proline, phenylalanine or tryptophan.

7. The vaccine of claim 6, wherein X₈ is isoleucine.

8. The vaccine of claim 1, wherein X₉ is H.

9. The vaccine of claim 1, wherein
X₁ is N;
X₂ is S;
X₃ is T;
X₄ is R; and
X₅ is S.

10. A vaccine comprising the sequence KKNSTYPTIKRSYNNTNQEDLLV[X]WGIHH (SEQ ID NO: 1) wherein the residue [X] is isoleucine, glycine, alanine, valine, proline, phenylalanine, tryptophan, serine, threonine, tyrosine, cysteine, asparagine or glutamine.

11. The vaccine of claim 10 wherein the residue [X] is isoleucine.

12. An antibody to the substantially isolated Replikin peptide of claim 1 or to an antigenic subsequence thereof.

## Patentansprüche

1. Impfstoff umfassend das im Wesentlichen isolierte Replikin-Peptid umfassend die Aminosäuresequenz KKX₁X₂X₃YPTIKX₄X₅X₆NNTNX₇EDLLVX₈WGIX₉H (SEQ ID NO: 349) oder eine antigene Teilsequenz davon, wobei
X₁ N oder G ist;
X₂ N oder S ist;
X₃ A oder T ist;
X₄ R oder K ist;
X₅ S oder T ist;
X₆ jeder Aminosäurerest ist;
X₇ jeder Aminosäurerest ist;
X₈ Isoleucin, Glycin, Alanin, Valin, Prolin, Phenylalanin, Tryptophan, Serin, Threonin, Tyrosin, Cystein, Asparagin oder Glutamin ist; und
X₉ H oder Q ist.

2. Impfstoff nach Anspruch 1, wobei X₆ Y ist.

3. Impfstoff nach Anspruch 1, wobei X₇ Q, I, M, V oder H ist.

4. Impfstoff nach Anspruch 1, wobei X₇ Q oder H ist.

5. Impfstoff nach Anspruch 4, wobei X₇ Q ist.

6. Impfstoff nach Anspruch 1, wobei X₈ Isoleucin, Glycin, Alanin, Valin, Prolin, Phenylalanin oder Tryptophan ist.

7. Impfstoff nach Anspruch 6, wobei X₈ Isoleucin ist.

8. Impfstoff nach Anspruch 1, wobei X₉ H ist.

9. Impfstoff nach Anspruch 1, wobei
X₁ N ist;
X₂ S ist;
X₃ T ist;
X₄ R ist; und
X₅ S ist.

10. Impfstoff umfassend die Sequenz KKNSTYPTIKRSYNNTNQEDLLV[X]WGIHH (SEQ ID NO: 1), wobei der Rest [X] Isoleucin, Glycin, Alanin, Valin, Prolin, Phenylalanin, Tryptophan, Serin, Threonin, Tyrosin, Cystein, Asparagin oder Glutamin ist.

11. Impfstoff nach Anspruch 10, wobei der Rest [X] Isoleucin ist.

12. Antikörper gegen das im Wesentlichen isolierten Replikin-Peptid nach Anspruch 1 oder zu einer antigenen Teilsequenz davon.

## Revendications

1. Vaccin comprenant le peptide Réplikine essentiellement isolé, comprenant la séquence d'acides aminés KKX₁X₂X₃YPTIKX₄X₅X₆NNTNX₇EDLLVX₈WGIX₉H (SEQ ID NO:349), ou une sous-séquence antigénique de cette dernière, dans laquelle
X₁ est N ou G ;
X₂ est N ou S ;
X₃ est A ou T ;
X₄ est R ou K ;
X₅ est S ou T ;
X₆ est un résidu d'acide aminé quelconque ;
X₇ est un résidu d'acide aminé quelconque ;
X₈ est une isoleucine, une glycine, une alanine, une valine, une proline, une phénylalanine, un tryptophane, une sérine, une thréonine, une tyrosine, une cystéine, une asparagine ou une glutamine ; et
X₉ est H ou Q.

2. Vaccin selon la revendication 1, dans lequel X₆ est Y.

3. Vaccin selon la revendication 1, dans lequel X₇ est Q, I, M, V ou H.

4. Vaccin selon la revendication 1, dans lequel X₇ est Q ou H.

5. Vaccin selon la revendication 4, dans lequel X₇ est Q.

6. Vaccin selon la revendication 1, dans lequel X₈ est une isoleucine, une glycine, une alanine, une valine, une proline, une phénylalanine ou un tryptophane.

7. Vaccin selon la revendication 6, dans lequel X₈ est une isoleucine.

8. Vaccin selon la revendication 1, dans lequel X₉ est H.

9. Vaccin selon la revendication 1, dans lequel
X₁ est N ;
X₂ est S ;
X₃ est T ;
X₄ est R ; et
X₅ est S.

10. Vaccin comprenant la séquence KKNSTYPTIKRSYNNTNQEDLLV[X]WGIHH (SEQ ID NO:1), dans lequel le résidu [X] est une isoleucine, une glycine, une alanine, une valine, une proline, une phénylalanine, un tryptophane, une sérine, une thréonine, une tyrosine, une cystéine, une asparagine ou une glutamine.

11. Vaccin selon la revendication 10, dans lequel le résidu [X] est une isoleucine.

12. Anticorps contre le peptide Réplikine essentiellement isolé de la revendication 1, ou contre une sous-séquence antigénique de ce dernier.
